# EUROPEAN PATENT APPLICATION

(11) **EP 3 895 722 A1**
(43) Date of publication of application: **20.10.2021**
(21) Application number: 21158079.0
(22) Date of filing: 06.01.2017
(51) Int. Cl.: A61K 38/07, C07K 5/10, C07K 5/11

(54) **METHODS FOR THE TREATMENT OF DUCHENNE MUSCULAR DYSTROPHY**

(30) Priority: 06.01.2016 US 201662275369 P
(62) Divisional of application: 17736433.8
(71) Applicant: Wilson, D. Travis, Newton, Massachusetts 02461 (US)
(72) Inventor: Wilson, D. Travis, Newton, Massachusetts 02461 (US)
(74) Representative: Barker Brettell LLP

(57) **Abstract**

The disclosure provides methods of preventing or treating DMD in a mammalian subject, reducing risk factors associated with DMD, and/or reducing the likelihood or severity of DMD. The methods comprise administering to the subject an effective amount of an aromatic-cationic peptide.

## Description

### CROSS-REFERENCE TO RELATED APPLICATIONS

This application claims priority to U.S. Provisional Patent Application No. 62/275,369, filed January 6, 2016, the content of which is hereby incorporated by reference in its entirety.

### TECHNICAL FIELD

The present technology relates generally to compositions and methods for preventing, ameliorating or treating Duchenne muscular dystrophy and/or reducing the severity of one or more risk factors, signs, or symptoms associated with Duchenne muscular dystrophy. Additionally, the present technology relates to administering an effective amount of an aromatic-cationic peptide, such as 2'6'-Dmt-D-Arg-Phe-Lys-NH₂, Phe-D-Arg-Phe-Lys-NH₂, or D-Arg-2'6'-Dmt-Lys-Phe-NH₂, to a subject suffering from or at risk for Duchenne muscular dystrophy.

### BACKGROUND

The following description is provided to assist the understanding of the reader. None of the information provided or references cited is admitted to be prior art to the compositions and methods disclosed herein.

Muscular dystrophy (MD) is a group of inherited non-inflammatory but progressive muscle disorders without a central or peripheral nerve abnormality. Duchenne muscular dystrophy (DMD) is the most common muscular dystrophy affecting 1 in 3500 young males born worldwide. DMD is caused by mutations in the dystrophin gene at locus Xp21, located on the short arm of the X chromosome. Dystrophin encodes a 427-kD protein that plays an integral role in the structural stability of the myofiber. Without dystrophin, muscles fibers are susceptible to mechanical injury, undergo necrosis and are ultimately replaced with adipose and connective tissue.

DMD is a progressive disease which eventually affects all voluntary muscles as well as cardiac and breathing muscles in later stages. Late-stage cardiac fibrosis can lead to output failure and pulmonary congestion, a common cause of death. Additionally, cardiac fibrosis can include cardiomyopathy and conduction abnormalities, which can induce fatal arrhythmias. The average life expectancy for individuals afflicted with DMD is around 25.

Although significant advances have been made in understanding the molecular underpinnings of the disorder, DMD remains an incurable illness.

### SUMMARY

In one aspect, the present disclosure provides methods for treating or preventing DMD, and/or treating or preventing the signs or symptoms of DMD in a subject in need thereof comprising administering to the subject a therapeutically effective amount of an aromatic-cationic peptide such as 2'6'-Dmt-D-Arg-Phe-Lys-NH₂, Phe-D-Arg-Phe-Lys-NH₂, or D-Arg-2'6'-Dmt-Lys-Phe-NH₂ or a pharmaceutically acceptable salt thereof, wherein the subject harbors a genetic alteration that disrupts the production or function of dystrophin protein. In some embodiments of the method, the genetic alteration is a deletion, duplication, frameshift, or nonsense mutation of dystrophin. In some embodiments of the methods of the present technology, the pharmaceutically acceptable salt comprises acetate, tartrate, or trifluoroacetate salt.

In some embodiments of the methods of the present technology, the signs or symptoms of DMD include one or more of progressive proximal weakness with onset in the legs and pelvis, hyperlordosis with wide-based gait, hypertrophy of weak muscles, pseudohypertrophy (enlargement of calf and deltoid muscles with fat and fibrotic tissue), reduced muscle contractility on electrical stimulation in advanced stages of the disease, delayed motor milestones, progressive inability to ambulate, heel cord contractures, paralysis, fatigue, skeletal deformities including scoliosis, muscle fiber deformities, cardiomyopathy, congestive heart failure or arrhythmia, muscular atrophy, respiratory disorders, and absence of bladder or bowel dysfunction, sensory disturbance, or febrile illness.

In some embodiments of the methods of the present technology, the subject displays elevated blood levels of creatine phosphokinase compared to a normal control subject. In certain embodiments of the methods of the present technology, treatment with the aromatic-cationic peptide normalizes creatine phosphokinase blood levels.

In some embodiments of the methods, administration of the aromatic-cationic peptide results in an increase in utrophin expression levels and/or activity compared to an untreated DMD control subject. In certain embodiments, administration of the aromatic-cationic peptide results in an increase in IGF-1 expression levels and/or activity compared to an untreated DMD control subject. In some embodiments of the methods, administration of the aromatic-cationic peptide results in an increase in follistatin expression levels and/or activity compared to an untreated DMD control subject.

In some embodiments of the methods, administration of the aromatic-cationic peptide results in an increase in Galgt2 expression levels and/or activity compared to an untreated DMD control subject. In some embodiments of the methods, administration of the aromatic-cationic peptide results in an increase in calpastatin expression levels and/or activity compared to an untreated DMD control subject. In some embodiments of the methods, administration of the aromatic-cationic peptide results in a decrease in calpain expression levels and/or activity compared to an untreated DMD control subject.

In some embodiments of the methods of the present technology, the subject is human.

In some embodiments of the methods of the present technology, the aromatic-cationic peptide is administered orally, topically, systemically, intravenously, subcutaneously, transdermally, iontophoretically, intranasally, intraperitoneally, or intramuscularly.

In some embodiments of the methods of the present technology, the aromatic-cationic peptide is administered daily for 1 week or more. In some embodiments of the methods of the present technology, the aromatic-cationic peptide is administered daily for 2 weeks or more. In some embodiments of the methods of the present technology, the aromatic-cationic peptide is administered daily for 3 weeks or more. In some embodiments of the methods of the present technology, the aromatic-cationic peptide is administered daily for 4 weeks or more. In some embodiments of the methods of the present technology, the aromatic-cationic peptide is administered daily for 6 weeks or more. In some embodiments of the methods of the present technology, the aromatic-cationic peptide is administered daily for 12 weeks or more.

In some embodiments, in addition to the administration of the aromatic-cationic peptide, the method further comprises separately, sequentially or simultaneously administering to the subject one or more additional therapeutic agents selected from the group consisting of: corticosteroids, Oxandrolone, ACE inhibitors, P188 (Poloxamer 188), beta-blockers, diuretics, angiotensin receptor blockers (ARBs), idebenone, alendronate, calcium with vitamin D, albuterol, dantrolene, pentoxifylline, carnitine, Coenzyme Q10, creatine, fish oil, green tea extracts, Vitamin E, PTC-124 (PTC Therapeutics Inc., South Plainfield, NJ), AVI-4658 phosphorodiamidate morpholino oligomer, azathioprine and cyclosporine.

In some embodiments, in addition to the administration of the aromatic-cationic peptide, the method further comprises separately, sequentially or simultaneously administering to the subject one or more ACE inhibitors (angiotensin II converting enzyme inhibitors) selected from the group consisting of captopril, alacepril, lisinopril, imidapril, quinapril, temocapril, delapril, benazepril, cilazapril, trandolapril, enalapril, ceronapril, fosinopril, imadapril, mobertpril, perindopril, ramipril, spirapril, randolapril and pharmaceutically acceptable salts of such compounds. In some embodiments of the methods of the present technology, there is a synergistic effect between the aromatic-cationic peptide and the ACE inhibitors with respect to the prevention or treatment of DMD.

In some embodiments, in addition to the administration of the aromatic-cationic peptide, the method further comprises separately, sequentially or simultaneously administering to the subject one or more ARBs selected from the group consisting of losartan, candesartan, valsartan, eprosartan, telmisartan, and irbesartan. In some embodiments of the methods of the present technology, there is a synergistic effect between the aromatic-cationic peptide and the ARBs with respect to the prevention or treatment of DMD.

In another aspect, the present technology provides a method for reducing progressive muscular dystrophy characterized by pseudohypertrophy in a mammalian subject having or suspected of having DMD, the method comprising: administering to the subject a therapeutically effective amount of the aromatic-cationic peptide 2'6'-Dmt-D-Arg-Phe-Lys-NH₂, Phe-D-Arg-Phe-Lys-NH₂, or D-Arg-2'6'-Dmt-Lys-Phe-NH₂ or a pharmaceutically acceptable salt thereof. In some embodiments of the methods of the present technology, the pharmaceutically acceptable salt comprises acetate, tartrate, or trifluoroacetate salt.

In some embodiments of the methods of the present technology, the mammalian subject shows reduced dystrophin protein levels in muscle fibers compared to a normal control subject. In certain embodiments of the methods of the present technology, the subject harbors a mutation in the dystrophin gene. In a further embodiment, the subject harbors a deletion, duplication, frameshift, or nonsense mutation in the dystrophin gene.

In some embodiments of the methods of the present technology, the mammalian subject has elevated blood levels of creatine phosphokinase compared to a normal control subj ect.

In some embodiments of the methods of the present technology, the subject is human. In some embodiments of the methods of the present technology, the aromatic-cationic peptide is administered orally, topically, systemically, intravenously, subcutaneously, transdermally, iontophoretically, intranasally, intraperitoneally, or intramuscularly.

In some embodiments of the methods, administration of the aromatic-cationic peptide results in an increase in utrophin expression levels and/or activity compared to an untreated DMD control subject. In certain embodiments, administration of the aromatic-cationic peptide results in an increase in IGF-1 expression levels and/or activity compared to an untreated DMD control subject. In some embodiments of the methods, administration of the aromatic-cationic peptide results in an increase in follistatin expression levels and/or activity compared to an untreated DMD control subject. In some embodiments of the methods, administration of the aromatic-cationic peptide results in an increase in Galgt2 expression levels and/or activity compared to an untreated DMD control subject. In some embodiments of the methods, administration of the aromatic-cationic peptide results in an increase in calpastatin expression levels and/or activity compared to an untreated DMD control subject. In some embodiments of the methods, administration of the aromatic-cationic peptide results in a decrease in calpain expression levels and/or activity compared to an untreated DMD control subject.

In one aspect, the present technology provides for methods for reducing the risk, signs or symptoms of DMD in a mammalian subject having decreased expression of dystrophin compared to a normal control subject. In some embodiments, the method includes administering to the subject a therapeutically effective amount of the aromatic-cationic peptide 2'6'-Dmt-D-Arg-Phe-Lys-NH₂, Phe-D-Arg-Phe-Lys-NH₂, or D-Arg-2'6'-Dmt-Lys-Phe-NH₂ or a pharmaceutically acceptable salt thereof, thereby resulting in the prevention or delay of onset of one or more risks, signs or symptoms of DMD. In some embodiments of the methods of the present technology, the pharmaceutically acceptable salt comprises acetate, tartrate, or trifluoroacetate salt.

In some embodiments of the methods of the present technology, the signs or symptoms of DMD include one or more of progressive proximal weakness with onset in the legs and pelvis, hyperlordosis with wide-based gait, hypertrophy of weak muscles, pseudohypertrophy (enlargement of calf and deltoid muscles with fat and fibrotic tissue), reduced muscle contractility on electrical stimulation in advanced stages of the disease, delayed motor milestones, progressive inability to ambulate, heel cord contractures, paralysis, fatigue, skeletal deformities including scoliosis, muscle fiber deformities, cardiomyopathy, congestive heart failure or arrhythmia, muscular atrophy, respiratory disorders, and absence of bladder or bowel dysfunction, sensory disturbance, or febrile illness.

In certain embodiments of the methods of the present technology, the subject harbors a mutation in the dystrophin gene. In some embodiments of the methods of the present technology, the subject is human.

In some embodiments of the methods of the present technology, the aromatic-cationic peptide is administered orally, topically, systemically, intravenously, subcutaneously, transdermally, iontophoretically, intranasally, intraperitoneally, or intramuscularly.

In some embodiments of the methods of the present technology, the aromatic-cationic peptide is administered daily for 1 week or more. In some embodiments of the methods of the present technology, the aromatic-cationic peptide is administered daily for 2 weeks or more. In some embodiments of the methods of the present technology, the aromatic-cationic peptide is administered daily for 3 weeks or more. In some embodiments of the methods of the present technology, the aromatic-cationic peptide is administered daily for 4 weeks or more. In some embodiments of the methods of the present technology, the aromatic-cationic peptide is administered daily for 6 weeks or more. In some embodiments of the methods of the present technology, the aromatic-cationic peptide is administered daily for 12 weeks or more.

In some embodiments, in addition to the administration of the aromatic-cationic peptide, the method further comprises separately, sequentially or simultaneously administering to the subject one or more additional therapeutic agents selected from the group consisting of: corticosteroids, Oxandrolone, ACE inhibitors, P188 (Poloxamer 188), beta-blockers, diuretics, angiotensin receptor blockers (ARBs), idebenone, alendronate, calcium with vitamin D, albuterol, dantrolene, pentoxifylline, carnitine, Coenzyme Q10, creatine, fish oil, green tea extracts, Vitamin E, PTC-124 (PTC Therapeutics Inc., South Plainfield, NJ), AVI-4658 phosphorodiamidate morpholino oligomer, azathioprine and cyclosporine.

In some embodiments, in addition to the administration of the aromatic-cationic peptide, the method further comprises separately, sequentially or simultaneously administering to the subject one or more ACE inhibitors (angiotensin II converting enzyme inhibitors) selected from the group consisting of captopril, alacepril, lisinopril, imidapril, quinapril, temocapril, delapril, benazepril, cilazapril, trandolapril, enalapril, ceronapril, fosinopril, imadapril, mobertpril, perindopril, ramipril, spirapril, randolapril and pharmaceutically acceptable salts of such compounds. In some embodiments of the methods of the present technology, there is a synergistic effect between the aromatic-cationic peptide and the ACE inhibitors with respect to reducing the risk, signs or symptoms of DMD.

In some embodiments, in addition to the administration of the aromatic-cationic peptide, the method further comprises separately, sequentially or simultaneously administering to the subject one or more ARBs selected from the group consisting of losartan, candesartan, valsartan, eprosartan, telmisartan, and irbesartan. In some embodiments of the methods of the present technology, there is a synergistic effect between the aromatic-cationic peptide and the ARBs with respect to reducing the risk, signs or symptoms of DMD.

Aspects or embodiments of the invention may also be provided according to the following paragraphs:
1. A method for treating or preventing DMD in a subject in need thereof, comprising administering to the subject a therapeutically effective amount of the peptide Phe-D-Arg-Phe-Lys-NH₂ or a pharmaceutically acceptable salt thereof, thereby resulting in the treatment or prevention of one or more signs or symptoms of DMD, wherein the subject harbors a genetic alteration that disrupts the production or function of dystrophin.
2. The method of paragraph 1, wherein the subject displays elevated blood levels of creatine phosphokinase compared to a normal control subject, and wherein peptide administration normalizes blood levels of creatine phosphokinase.
3. The method of any one of paragraphs 1-2, wherein the peptide is administered daily for 6 weeks or more.
4. The method of any one of paragraphs 1-2, wherein the peptide is administered daily for 12 weeks or more.
5. The method of any one of paragraphs 1-4, wherein the subject has been diagnosed as having DMD.
6. The method of paragraph 5, wherein the signs or symptoms of DMD comprise one or more of progressive proximal weakness with onset in the legs and pelvis, hyperlordosis with wide-based gait, hypertrophy of weak muscles, pseudohypertrophy (enlargement of calf and deltoid muscles with fat and fibrotic tissue), reduced muscle contractility on electrical stimulation in advanced stages of the disease, delayed motor milestones, progressive inability to ambulate, heel cord contractures, paralysis, fatigue, skeletal deformities including scoliosis, muscle fiber deformities, cardiomyopathy, congestive heart failure or arrhythmia, muscular atrophy, and respiratory disorders.
7. The method of any one of paragraphs 1-6, wherein the subject is human.
8. The method of any one of paragraphs 1-7, wherein the peptide is administered orally, topically, systemically, intravenously, subcutaneously, transdermally, iontophoretically, intranasally, intraperitoneally, or intramuscularly.
9. The method of any one of paragraphs 1-8, further comprising separately, sequentially or simultaneously administering an additional therapeutic agent to the subject.
10. The method of paragraph 9, wherein the additional therapeutic agent is selected from the group consisting of: corticosteroids, Oxandrolone, ACE inhibitors, P188 (Poloxamer 188), beta-blockers, diuretics, angiotensin receptor blockers (ARBs), idebenone, alendronate, calcium with vitamin D, albuterol, dantrolene, pentoxifylline, carnitine, Coenzyme Q10, creatine, fish oil, green tea extracts, Vitamin E, PTC-124, AVI-4658 phosphorodiamidate morpholino oligomer, azathioprine and cyclosporine.
11. The method of paragraph 10, wherein the combination of the peptide and the additional therapeutic agent has a synergistic effect in the prevention or treatment of DMD.
12. The method of any one of paragraphs 1-11, wherein the pharmaceutically acceptable salt comprises acetate, tartrate or trifluoroacetate salt.
13. The method of any one of paragraphs 1-12, wherein peptide administration results in an increase in the expression levels and/or activity of one or more of utrophin, IGF-1, follistatin, Galgt2, and calpastatin compared to an untreated DMD control subject.
14. The method of any one of paragraphs 1-13, wherein peptide administration results in a decrease in calpain expression levels and/or activity compared to an untreated DMD control subject.
15. A method for reducing the risk of DMD in a subject in need thereof, the method comprising: administering to the subject a therapeutically effective amount of the peptide Phe-D-Arg-Phe-Lys-NH₂ or a pharmaceutically acceptable salt thereof, thereby resulting in the prevention or delay of onset of one or more signs or symptoms of DMD.
16. The method of paragraph 15, wherein the subject harbors a mutation in the dystrophin gene.
17. The method of any one of paragraphs 15-16, wherein the peptide is administered daily for 6 weeks or more.
18. The method of any one of paragraphs 15-16, wherein the peptide is administered daily for 12 weeks or more.
19. The method of any one of paragraphs 15-18, wherein the subject is human.
20. The method of any one of paragraphs 15-19, wherein the peptide is administered orally, topically, systemically, intravenously, subcutaneously, transdermally, iontophoretically, intranasally, intraperitoneally, or intramuscularly.
21. The method of any one of paragraphs 15-20, further comprising separately, sequentially or simultaneously administering the additional therapeutic agent to the subject.
22. The method of paragraph 21, wherein the additional therapeutic agent is selected from the group consisting of: corticosteroids, Oxandrolone, ACE inhibitors, P188 (Poloxamer 188), beta-blockers, diuretics, angiotensin receptor blockers (ARBs), idebenone, alendronate, calcium with vitamin D, albuterol, dantrolene, pentoxifylline, carnitine, Coenzyme Q10, creatine, fish oil, green tea extracts, Vitamin E, PTC-124, AVI-4658 phosphorodiamidate morpholino oligomer, azathioprine and cyclosporine.
23. The method of paragraph 22, wherein the combination of the peptide and the additional therapeutic agent has a synergistic effect in reducing the risk of DMD.
24. The method of any one of paragraphs 15-23, wherein the pharmaceutically acceptable salt comprises acetate, tartrate or trifluoroacetate salt.
25. A method for reducing progressive muscular dystrophy characterized by pseudohypertrophy in a mammalian subject having or suspected of having DMD, the method comprising: administering to the subject a therapeutically effective amount of Phe-D-Arg-Phe-Lys-NH₂, or a pharmaceutically acceptable salt thereof.
26. The method of paragraph 25, wherein the pharmaceutically acceptable salt comprises acetate, tartrate, or trifluoroacetate salt.
27. The method of any one of paragraphs 25-26, wherein the mammalian subject shows reduced dystrophin protein levels in muscle fibers compared to a normal control subject.
28. The method of any one of paragraphs 25-27, wherein the subject harbors a deletion, duplication, frameshift, or nonsense mutation in the dystrophin gene.
29. The method of any one of paragraphs 25-28, wherein the mammalian subject has elevated blood levels of creatine phosphokinase compared to a normal control subject.
30. The method of any one of paragraphs 25-29, wherein the subject is human.
31. The method of any one of paragraphs 25-30, wherein the aromatic-cationic peptide is administered orally, topically, systemically, intravenously, subcutaneously, transdermally, iontophoretically, intranasally, intraperitoneally, or intramuscularly.
32. The method of any one of paragraphs 25-31, wherein administration of the aromatic-cationic peptide results in an increase in the expression levels and/or activity of one or more of utrophin, IGF-1, follistatin, Galgt2, and calpastatin compared to an untreated DMD control subject.
33. The method of any one of paragraphs 25-32, wherein administration of the aromatic-cationic peptide results in a decrease in calpain expression levels and/or activity compared to an untreated DMD control subject.

### DETAILED DESCRIPTION

It is to be appreciated that certain aspects, modes, embodiments, variations and features of the present technology are described below in various levels of detail in order to provide a substantial understanding of the present technology. The definitions of certain terms as used in this specification are provided below. Unless defined otherwise, all technical and scientific terms used herein generally have the same meaning as commonly understood by one of ordinary skill in the art to which this present technology belongs.

As used in this specification and the appended claims, the singular forms "a", "an" and "the" include plural referents unless the content clearly dictates otherwise. For example, reference to "a cell" includes a combination of two or more cells, and the like.

As used herein, the "administration" of an agent, drug, or peptide to a subject includes any route of introducing or delivering to a subject a compound to perform its intended function. Administration can be carried out by any suitable route, including orally, intranasally, parenterally (intravenously, intramuscularly, intraperitoneally, or subcutaneously), or topically. Administration includes self-administration and the administration by another.

As used herein, the term "amino acid" includes naturally-occurring amino acids and synthetic amino acids, as well as amino acid analogs and amino acid mimetics that function in a manner similar to the naturally-occurring amino acids. Naturally-occurring amino acids are those encoded by the genetic code, as well as those amino acids that are later modified, *e.g.,* hydroxyproline, γ-carboxyglutamate, and O-phosphoserine. Amino acid analogs refers to compounds that have the same basic chemical structure as a naturally-occurring amino acid, *i.e*., an α-carbon that is bound to a hydrogen, a carboxyl group, an amino group, and an R group, *e.g.,* homoserine, norleucine, methionine sulfoxide, methionine methyl sulfonium. Such analogs have modified R groups (*e.g.,* norleucine) or modified peptide backbones, but retain the same basic chemical structure as a naturally-occurring amino acid. Amino acid mimetics refers to chemical compounds that have a structure that is different from the general chemical structure of an amino acid, but that functions in a manner similar to a naturally-occurring amino acid. Amino acids can be referred to herein by either their commonly known three letter symbols or by the one-letter symbols recommended by the IUPAC-IUB Biochemical Nomenclature Commission.

As used herein, the term "effective amount" refers to a quantity sufficient to achieve a desired therapeutic and/or prophylactic effect, *e.g.,* an amount which results in partial or full amelioration of one or more symptoms of DMD. In the context of therapeutic or prophylactic applications, in some embodiments, the amount of a composition administered to the subject will depend on the type, degree, and severity of the disease and on the characteristics of the individual, such as general health, age, sex, body weight and tolerance to drugs. The skilled artisan will be able to determine appropriate dosages depending on these and other factors. The compositions can also be administered in combination with one or more additional therapeutic compounds. In the methods described herein, aromatic-cationic peptides, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, hydrochloride salt, or trifluoroacetate salt, may be administered to a subject having one or more signs, symptoms, or risk factors of DMD, including, but not limited to, progressive proximal weakness with onset in the legs and pelvis, hyperlordosis with wide-based gait, hypertrophy of weak muscles, pseudohypertrophy (enlargement of calf and deltoid muscles with fat and fibrotic tissue), reduced muscle contractility on electrical stimulation in advanced stages of the disease, delayed motor milestones, progressive inability to ambulate, heel cord contractures, paralysis, fatigue, skeletal deformities including scoliosis, muscle fiber deformities, cardiomyopathy, congestive heart failure or arrhythmia, muscular atrophy, respiratory disorders, and absence of bladder or bowel dysfunction, sensory disturbance, or febrile illness. For example, a "therapeutically effective amount" of the aromatic-cationic peptides includes levels at which the presence, frequency, or severity of one or more signs, symptoms, or risk factors of DMD are, at a minimum, ameliorated. In some embodiments, a therapeutically effective amount reduces or ameliorates the physiological effects of DMD, and/or the risk factors of DMD, and/or the likelihood of developing DMD. A therapeutically effective amount can be given in one or more administrations.

As used herein, "isolated" or "purified" polypeptide or peptide refers to a polypeptide or peptide that is substantially free of cellular material or other contaminating polypeptides from the cell or tissue source from which the agent is derived, or substantially free from chemical precursors or other chemicals when chemically synthesized. For example, an isolated aromatic-cationic peptide would be free of materials that would interfere with diagnostic or therapeutic uses of the agent. Such interfering materials may include enzymes, hormones and other proteinaceous and nonproteinaceous solutes.

As used herein, the terms "polypeptide," "peptide," and "protein" are used interchangeably herein to mean a polymer comprising two or more amino acids joined to each other by peptide bonds or modified peptide bonds, *i.e.,* peptide isosteres. Polypeptide refers to both short chains, commonly referred to as peptides, glycopeptides or oligomers, and to longer chains, generally referred to as proteins. Polypeptides may contain amino acids other than the 20 gene-encoded amino acids. Polypeptides include amino acid sequences modified either by natural processes, such as post-translational processing, or by chemical modification techniques that are well known in the art.

As used herein, the terms "subject" and "patient" are used interchangeably.

As used herein, the term "simultaneous" therapeutic use refers to the administration of at least two active ingredients by the same route and at the same time or at substantially the same time.

As used herein, the term "separate" therapeutic use refers to an administration of at least two active ingredients at the same time or at substantially the same time by different routes.

As used herein, the term "sequential" therapeutic use refers to administration of at least two active ingredients at different times, the administration route being identical or different. More particularly, sequential use refers to the whole administration of one of the active ingredients before administration of the other or others commences. It is thus possible to administer one of the active ingredients over several minutes, hours, or days before administering the other active ingredient or ingredients. There is no simultaneous treatment in this case.

A "synergistic therapeutic effect" refers to a greater-than-additive therapeutic effect which is produced by a combination of at least two therapeutic agents, and which exceeds that which would otherwise result from the individual administration of the agents. For example, lower doses of one or more therapeutic agents may be used in treating DMD, resulting in increased therapeutic efficacy and decreased side-effects.

"Treating" or "treatment" as used herein covers the treatment of DMD, in a subject, such as a human, and includes: (i) inhibiting DMD, *i.e*., arresting its development; (ii) relieving DMD, *i.e*., causing regression of the disorder; (iii) slowing the progression of DMD; and/or (iv) inhibiting, relieving, or slowing progression of one or more symptoms of DMD.

As used herein, "preventing" or "prevention" of a disorder or condition refers to a compound that, in a statistical sample, reduces the occurrence of the disorder or condition in the treated sample relative to an untreated control sample, or delays the onset of one or more symptoms of the disorder or condition relative to the untreated control sample. As used herein, preventing DMD includes preventing or delaying the initiation of DMD. As used herein, prevention of DMD also includes preventing a recurrence of one or more signs or symptoms of DMD.

It is also to be appreciated that the various modes of treatment or prevention of medical conditions as described herein are intended to mean "substantial," which includes total but also less than total treatment or prevention, and wherein some biologically or medically relevant result is achieved. The treatment may be a continuous prolonged treatment for a chronic disease or a single, or few time administrations for the treatment of an acute condition.

### Aromatic-Cationic Peptides

The aromatic-cationic peptides of the present technology preferably include a minimum of three amino acids, covalently joined by peptide bonds.

The maximum number of amino acids present in the aromatic-cationic peptides of the present technology is about twenty amino acids covalently joined by peptide bonds. In some embodiments, the total number of amino acids is about twelve. In some embodiments, the total number of amino acids is about nine. In some embodiments, the total number of amino acids is about six. In some embodiments, the total number of amino acids is four.

In some aspects, the present technology provides an aromatic-cationic peptide or a pharmaceutically acceptable salt thereof such as acetate salt, tartrate salt, fumarate salt, hydrochloride salt, or trifluoroacetate salt. In some embodiments, the peptide comprises at least one net positive charge; a minimum of three amino acids; a maximum of about twenty amino acids;
a relationship between the minimum number of net positive charges (pₘ) and the total number of amino acid residues (r) wherein 3pₘ is the largest number that is less than or equal to r + 1; and
a relationship between the minimum number of aromatic groups (a) and the total number of net positive charges (pₜ) wherein 2a is the largest number that is less than or equal to pₜ + 1, except that when a is 1, pₜ may also be 1.

In some embodiments, the peptide is defined by Formula I: 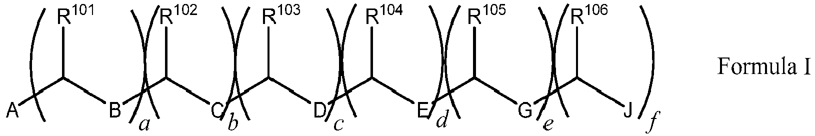 wherein:
one of A and J is 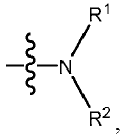 and the other of A and J is 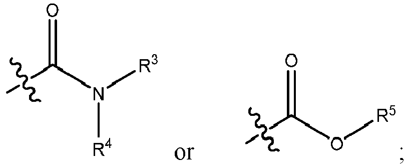
B, C, D, E, and G are each 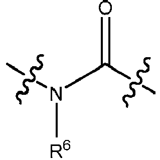 or B, C, D, E, and G are each 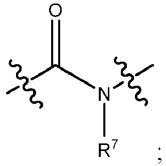 with the proviso that when
   f is 0 and J is not a terminal group, the terminal group is one of G, E, D or C, such that
   one of A and the terminal group is 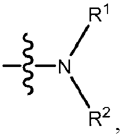 and
   the other of A and the terminal group is 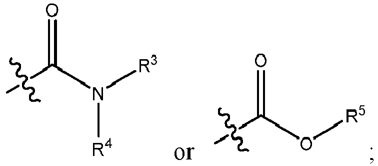
R¹⁰¹ is 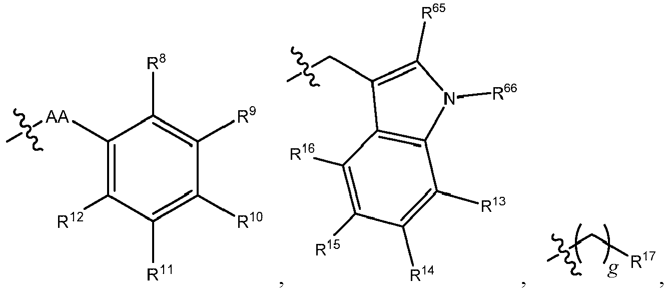 or 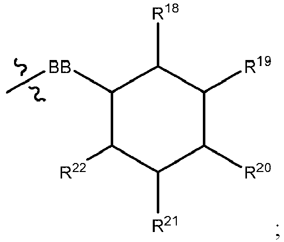
R¹⁰² is 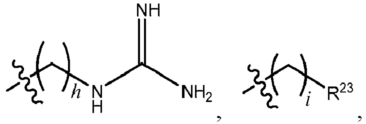 or hydrogen;
R¹⁰² is 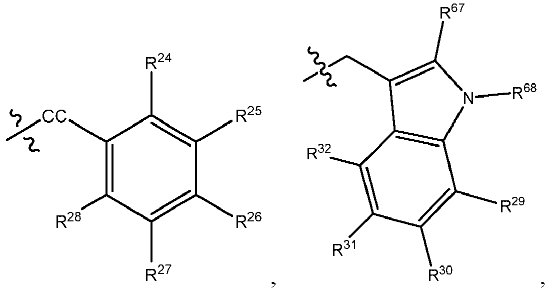 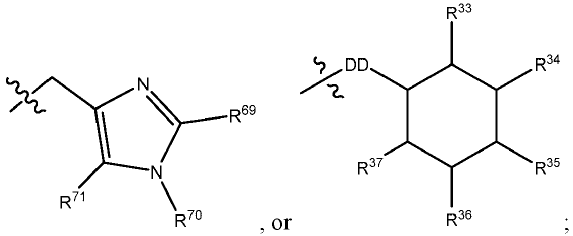
R¹⁰² is 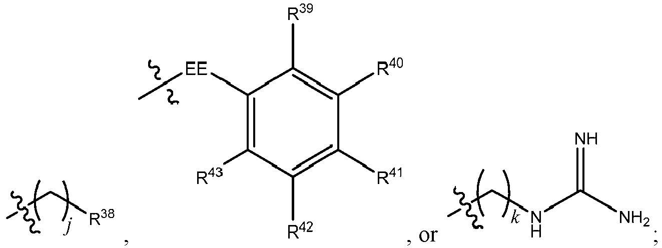
R¹⁰² is 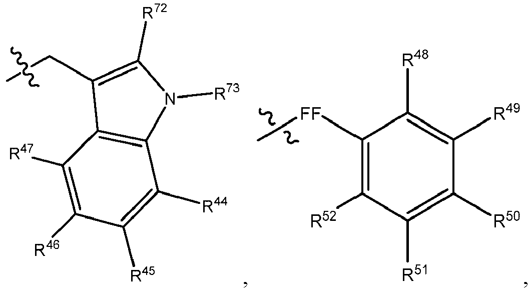 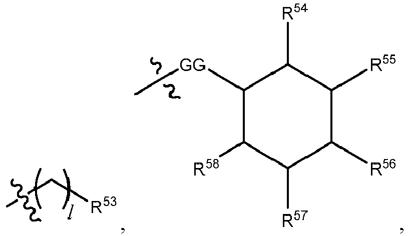 or hydrogen;
R¹⁰⁶ is 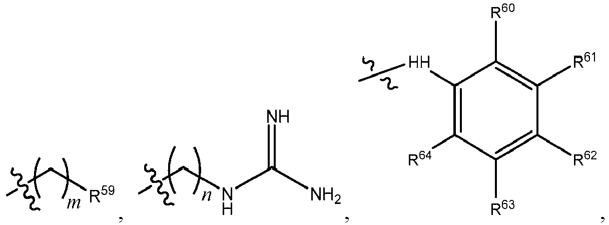 or hydrogen; provided that when R¹⁰², R¹⁰⁴, and R¹⁰⁶ are identical, then R¹⁰¹, R¹⁰³, and R¹⁰⁵ are not identical;
   wherein
   R¹, R², R³, R⁴, and R⁵ are each independently a hydrogen or substituted or unsubstituted C₁-C₆ alkyl, C₂-C₆ alkenyl, C₂-C₆ alkynyl, saturated or unsaturated cycloalkyl, cycloalkylalkyl, aryl, aralkyl, 5- or 6- membered saturated or unsaturated heterocylyl, heteroaryl, or amino protecting group; or R¹ and R² together form a 3, 4, 5, 6, 7, or 8 membered substituted or unsubstituted heterocycyl ring;
   R⁶ and R⁷ at each occurrence are independently a hydrogen or substituted or unsubstituted C₁-C₆ alkyl group;
   R⁸, R⁹, R¹⁰, R¹¹, R¹², R¹³, R¹⁴, R¹⁵, R¹⁶, R¹⁸, R¹⁹, R²⁰, R²¹, R²², R²⁴, R²⁵, R²⁶, R²⁷, R²⁸, R²⁹, R³⁰, R³¹, R³², R³³, R³⁴, R³⁵, R³⁶, R³⁷, R³⁹, R⁴⁰, R⁴¹, R⁴², R⁴³, R⁴⁴, R⁴⁵, R⁴⁶, R⁴⁷, R⁴⁸, R⁴⁹, R⁵⁰, R⁵¹, R⁵², R⁵⁴, R⁵⁵, R⁵⁶, R⁵⁷, R⁵⁸, R⁶⁰, R⁶¹, R⁶², R⁶³, R⁶⁴, R⁶⁵, R⁶⁷, R⁶⁹, R⁷¹, and R⁷² are each independently a hydrogen, amino, amido, -NO₂, - CN, -OR^{a}, -SR^{a}, -NR^{a}R^{a}, -F, -Cl, -Br, -I, or a substituted or unsubstituted C₁-C₆ alkyl, C₁-C₆ alkoxy, -C(O)-alkyl, -C(O)-aryl, - C(O)-aralkyl, -C(O)₂R^{a}, C₁-C₄ alkylamino, C₁-C₄ dialkylamino, or perhaloalkyl group;
   R⁶⁶, R⁶⁸, R⁷⁰, and R⁷³ are each independently a hydrogen or substituted or unsubstituted C₁-C₆ alkyl group;
   R¹⁷, R²³, R³⁸, R⁵³, and R⁵⁹ are each independently a hydrogen, -OR^{a}, - SR^{a}, -NR^{a}R^{a}, -NR^{a}R^{b}, -CO₂R^{a}, -(CO)NR^{a}R^{a}, -NR^{a}(CO)R^{a}, -NR^{a}C(NH)NH₂, -NR^{a}-dansyl, or a substituted or unsubstituted alkyl, aryl, or aralkyl group;
   AA, BB, CC, DD, EE, FF, GG, and HH are each independently absent, -NH(CO)-, or -CH₂-;
   R^{a} at each occurrence is independently a hydrogen or a substituted or unsubstituted C₁-C₆ alkyl group;
   R^{b} at each occurrence is independently a C₁-C₆ alkylene-NR^{a}-dansyl or C₁-C₆ alkylene-NR^{a}-anthraniloyl group;
   *a, b, c, d, e,* and *f* are each independently 0 or 1,
      with the proviso that *a* + *b* + *c* + *d* + *e* + *f* ≥ 2;
   *g, h, k, m,* and *n* are each independently 1, 2, 3, 4, or 5; and
   *i, j,* and *l* are each independently 2, 3, 4, or 5;
   provided that
      when *i* is 4 and R²³ is -SR^{a}, *or j* is 4 and R³⁸ is -SR^{a}, or *l* is 4 and R⁵³ is -SR^{a}, then the R^{a} of the -SR^{a} is a substituted or unsubstituted C₁-C₆ alkyl group;
      when J is -NH₂, *b* and *d* are 0, *a, c, e, f* are 1, then R¹⁰³ is

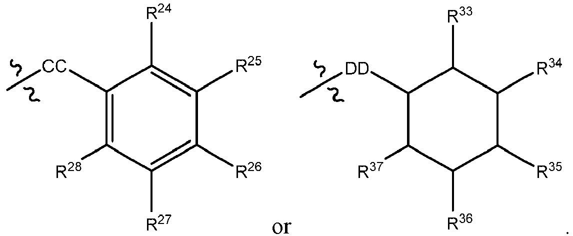

In some embodiments of peptides of Formula I,
R¹, R², R³, R⁴, and R⁵ are each independently a hydrogen or substituted or unsubstituted C₁-C₆ alkyl group;
R⁶ and R⁷ at each occurrence are independently a hydrogen or methyl group;
R⁸, R¹², R¹⁸, R²², R²⁴, R²⁸, R³³, R³⁷, R³⁹, R⁴³, R⁴⁸, R⁵², R⁵⁴, R⁵⁸, R⁶¹, and R⁶⁴ are each independently a hydrogen or methyl group;
R¹⁰, R²⁰, R²⁶, R³⁵, R⁴¹, R⁵⁰, R⁵⁶, and R⁶² are each independently a hydrogen or -OR^{a};
R⁹, R¹¹, R¹⁹, R²¹, R²⁵, R²⁷, R³⁴, R³⁶, R⁴⁰, R⁴², R⁴⁹, R⁵¹, R⁵⁵, R⁵⁷, R⁶¹, R⁶³, R⁶⁵, R⁶⁶, R⁶⁷, R⁶⁸, R⁶⁹, R⁷⁰, R⁷¹, R⁷², and R⁷³ are each a hydrogen;
R¹⁷, R²³, R³⁸, R⁵³, and R⁵⁹ are each independently a hydrogen, -OH, -SH, -SCH₃, - NH₂, -NHR^{b}, -CO₂H, -(CO)NH₂, -NH(CO)H, or -NH-dansyl group;
AA, BB, CC, DD, EE, FF, GG, and HH are each independently absent or -CH₂-;
R^{a} at each occurrence is independently a hydrogen or a substituted or unsubstituted C₁-C₆ alkyl group;
R^{b} at each occurrence is independently an ethylene-NH-dansyl or ethylene-NH-anthraniloyl group.

In some embodiments of Formula I,
A is 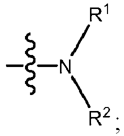
J is 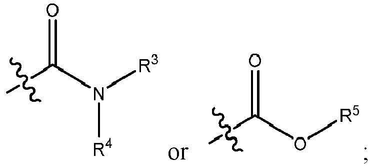
B, C, D, E, and G are each independently 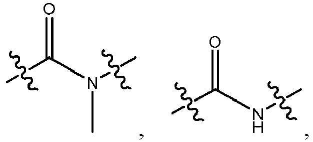 or absent;
   with the proviso when *f* is 0, G is 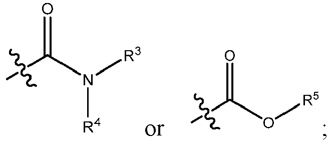
   when *e* and *f* are 0, E is 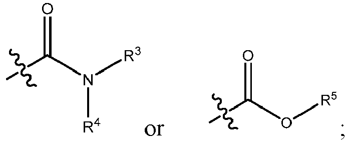
   when *d, e,* and *f* are 0, D is 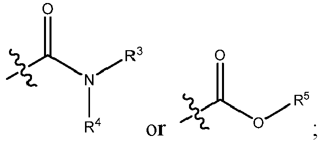 and
   when *c, d, e,* and *f* are 0, C is

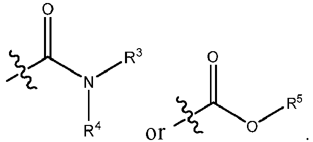

In another embodiment of Formula I,
A is 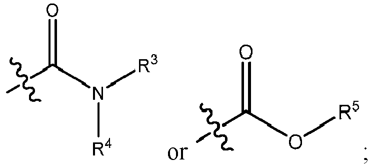
J is 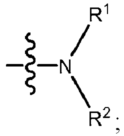
B, C, D, E, and G are each independently 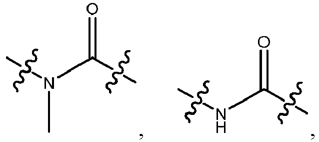 or absent;
   with the proviso when *f* is 0, G is 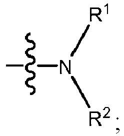
   when *e* and *f* are 0, E is 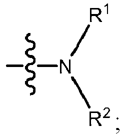
   when *d, e,* and *f* are 0, D is 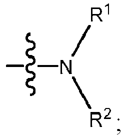 and
   when *c, d, e,* and *f* are 0, C is

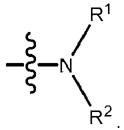

In some embodiments of Formula I, at least one of R¹⁰¹, R¹⁰², R¹⁰⁴, R¹⁰⁵, and Ris a basic group, as defined above, and at least one of R¹⁰¹, R¹⁰³, R¹⁰⁴, R¹⁰⁵, and R¹⁰⁶ is a neutral group as defined above. In some such embodiments, the neutral group is an aromatic, heterocyclic or cycloalkyl group as defined above. In some embodiments of Formula I, the peptide contains at least one arginine, such as, but not limited to D-arginine, and at least one 2',6'-dimethyltyrosine, tyrosine, or phenylalanine. In some embodiments of Formula I, R¹⁰¹ is an alkylguanidinium group.

In some embodiments, the peptide of the present technology is selected from the peptides shown in Tables A or B.

| **TABLE A** |
|---|
| Tyr-D-Arg-Phe-Lys-NH₂ |
| D-Arg-Dmt-Lys-Phe-NH₂ |
| D-Arg-Dmt-Phe-Lys-NH₂ |
| D-Arg-Phe-Lys-Dmt-NH₂ |
| D-Arg-Phe-Dmt-Lys-NH₂ |
| D-Arg-Lys-Dmt-Phe-NH₂ |
| D-Arg-Lys-Phe-Dmt-NH₂ |
| D-Arg-Dmt-Lys-Phe-Cys-NH₂ |
| Phe-Lys-Dmt-D-Arg-NH₂ |
| Phe-Lys-D-Arg-Dmt-NH₂ |
| Phe-D-Arg-Phe-Lys-NH₂ |
| Phe-D-Arg-Phe-Lys-Cys-NH₂ |
| Phe-D-Arg-Phe-Lys-Ser-Cys-NH₂ |
| Phe-D-Arg-Phe-Lys-Gly-Cys-NH₂ |
| Phe-D-Arg-Dmt-Lys-NH₂ |
| Phe-D-Arg-Dmt-Lys-Cys-NH₂ |
| Phe-D-Arg-Dmt-Lys-Ser-Cys-NH₂ |
| Phe-D-Arg-Dmt-Lys-Gly-Cys-NH₂ |
| Phe-D-Arg-Lys-Dmt-NH₂ |
| Phe-Dmt-D-Arg-Lys-NH₂ |
| Phe-Dmt-Lys-D-Arg-NH₂ |
| Lys-Phe-D-Arg-Dmt-NH₂ |
| Lys-Phe-Dmt-D-Arg-NH₂ |
| Lys-Dmt-D-Arg-Phe-NH₂ |
| Lys-Dmt-Phe-D-Arg-NH₂ |
| Lys-D-Arg-Phe-Dmt-NH₂ |
| Lys-D-Arg-Dmt-Phe-NH₂ |
| D-Arg-Dmt-D-Arg-Phe-NH₂ |
| D-Arg-Dmt-D-Arg-Dmt-NH₂ |
| D-Arg-Dmt-D-Arg-Tyr-NH₂ |
| D-Arg-Dmt-D-Arg-Trp-NH₂ |
| Trp-D-Arg-Tyr-Lys-NH₂ |
| Trp-D-Arg-Trp-Lys-NH₂ |
| Trp-D-Arg-Dmt-Lys-NH₂ |
| D-Arg-Trp-Lys-Phe-NH₂ |
| D-Arg-Trp-Phe-Lys-NH₂ |
| D-Arg-Trp-Lys-Dmt-NH₂ |
| D-Arg-Trp-Dmt-Lys-NH₂ |
| D-Arg-Lys-Trp-Phe-NH₂ |
| D-Arg-Lys-Trp-Dmt-NH₂ |
| Cha-D-Arg-Phe-Lys-NH₂ |
| Ala-D-Arg-Phe-Lys-NH₂ |
| 2',6'-Dmp-D-Arg-2',6'-Dmt-Lys-NH₂ |
| 2',6'-Dmp-D-Arg-Phe-Lys-NH₂ |
| 2',6'-Dmt-D-Arg-Phe-Orn-NH₂ |
| 2',6'-Dmt-D-Arg-Phe-Ahp-NH₂ |
| 2',6'-Dmt-D-Arg-Phe-Lys-NH₂ |
| 2',6'-Dmt-D-Cit-Phe-Lys-NH₂ |
| D-Arg-2',6'-Dmt-Lys-Phe-NH₂ |
| D-Tyr-Trp-Lys-NH₂ |
| Lys-D-Arg-Tyr-NH₂ |
| Met-Tyr-D-Arg-Phe-Arg-NH₂ |
| Met-Tyr-D-Lys-Phe-Arg |
| Phe-Arg-D-His-Asp |
| Phe-D-Arg-2',6'-Dmt-Lys-NH₂ |
| Phe-D-Arg-His |
| Trp-D-Lys-Tyr-Arg-NH₂ |
| Tyr-D-Arg-Phe-Lys-Glu-NH₂ |
| Tyr-His-D-Gly-Met |
| D-Arg-Tyr-Lys-Phe-NH₂ |
| D-Arg-*D-*Dmt-Lys- Phe-NH₂ |
| D-Arg-Dmt-*D*-Lys-Phe-NH₂ |
| D-Arg-Dmt-Lys-*D*-Phe-NH₂ |
| D-Arg-*D*-Dmt-*D*-Lys-*D*-Phe-NH₂ |
| Phe-*D*-Arg-*D*-Phe-Lys-NH₂ |
| Phe-*D*-Arg-Phe-*D*-Lys-NH₂ |
| D-Phe-*D*-Arg-*D*-Phe-*D*-Lys-NH₂ |
| Lys-*D*-Phe-Arg-Dmt-NH₂ |
| *D*-Arg-Arg-Dmt-Phe-NH₂ |
| Dmt-*D*-Phe -Arg-Lys-NH₂ |
| Phe-*D*-Dmt-Arg-Lys-NH₂ |
| *D*-Arg-Dmt-Lys-NH₂ |
| Arg-*D*-Dmt-Lys-NH₂ |
| *D*-Arg-Dmt-Phe-NH₂ |
| Arg-D-Dmt-Arg-NH₂ |
| Dmt-D-Arg-NH₂ |
| D-Arg-Dmt-NH₂ |
| D-Dmt-Arg-NH₂ |
| Arg-D-Dmt-NH₂ |
| D-Arg-D-Dmt-NH₂ |
| D-Arg-*D*-Tyr-Lys-Phe-NH₂ |
| D-Arg-Tyr-*D*-Lys-Phe-NH₂ |
| D-Arg-Tyr-Lys-D-Phe-NH₂ |
| D-Arg-*D*-Tyr-*D*-Lys-*D*-Phe-NH₂ |
| Lys-*D*-Phe-Arg-Tyr-NH₂ |
| *D*-Arg-Arg-Tyr-Phe-NH₂ |
| Tyr-*D*-Phe-Arg-Lys-NH₂ |
| Phe-*D*-Tyr-Arg-Lys-NH₂ |
| *D*-Arg-Tyr-Lys-NH₂ |
| Arg-*D*-Tyr-Lys-NH₂ |
| *D*-Arg-Tyr-Phe-NH₂ |
| Arg-*D*-Tyr-Arg-NH₂ |
| Tyr-*D*-Arg-NH₂ |
| *D*-Arg-Tyr-NH₂ |
| *D*-Tyr-Arg-NH₂ |
| Arg-*D*-Tyr-NH₂ |
| *D*-Arg-*D*-Tyr-NH₂ |
| Dmt-Lys-Phe-NH₂ |
| Lys-Dmt-*D*-Arg-NH₂ |
| Phe-Lys-Dmt-NH₂ |
| *D*-Arg-Phe-Lys-NH₂ |
| *D*-Arg-Cha-Lys-NH₂ |
| *D*-Arg-Trp-Lys-NH₂ |
| Dmt-Lys-*D*-Phe-NH₂ |
| Dmt-Lys-NH₂ |
| Lys-Phe-NH₂ |
| *D*-Arg-Cha-Lys-Cha-NH₂ |
| *D*-Nle-Dmt-Ahp-Phe-NH₂ |
| *D*-Nle-Cha-Ahp-Cha-NH₂ |
| D-Arg-Dmt-D-Lys-NH₂ |
| D-Arg-Dmt-D-Lys-Phe-NH₂ |
| Lys-Trp-D-Arg-NH₂ |
| H-Lys-D-Phe-Arg-Dmt-NH₂ |
| H-D-Arg-Lys-Dmt-Phe-NH₂ |
| H-D-Arg-Lys-Phe-Dmt-NH₂ |
| H-D-Arg-Arg-Dmt-Phe-NH₂ |
| H-D-Arg-Dmt-Phe-Lys-NH₂ |
| H-D-Arg-Phe-Dmt-Lys-NH₂ |
| H-Dmt-D-Phe-Arg-Lys-NH₂ |
| H-Phe-D-Dmt-Arg-Lys-NH₂ |
| H-D-Arg-Dmt-Lys-NH₂ |
| H-D-Arg-Dmt-D-Lys-D-Phe-NH₂ |
| H-D-Arg-D-Dmt-Lys-Phe-NH₂ |
| H-D-Arg-Dmt-Phe-NH₂ |
| H-Dmt-D-Arg-NH₂ |
| H-Phe-D-Arg-D-Phe-Lys-NH₂ |
| H-Phe-D-Arg-Phe-D-Lys-NH₂ |
| H-D-Phe-D-Arg-D-Phe-D-Lys-NH₂ |
| H-D-Arg-D-Dmt-D-Lys-D-Phe-NH₂ |
| H-D-Arg-Cha-Lys-NH₂ |
| H-D-Arg-Cha-Lys-Cha-NH₂ |
| H-Arg-D-Dmt-Lys-NH₂ |
| H-Arg-D-Dmt-Arg-NH₂ |
| H-D-Dmt-Arg-NH₂ |
| H-Arg-D-Dmt-NH₂ |
| H-D-Arg-D-Dmt-NH₂ |
| Arg-Arg-Dmt-Phe |
| Arg-Cha-Lys |
| Arg-Dmt |
| Arg-Dmt- Arg |
| Arg-Dmt-Lys |
| Arg-Dmt-Lys-Phe |
| Arg-Dmt-Lys-Phe-Cys |
| Arg-Dmt-Phe |
| Arg-Dmt-Phe-Lys |
| Arg-Lys-Dmt-Phe |
| Arg-Lys-Phe-Dmt |
| Arg-Phe-Dmt-Lys |
| Arg-Phe-Lys |
| Arg-Trp-Lys |
| Arg-Tyr-Lys |
| Arg-Tyr-Lys-Phe |
| D-Arg-D-Dmt-D-Lys-L-Phe-NH₂ |
| D-Arg-D-Dmt-L-Lys-D-Phe-NH₂ |
| D-Arg-D-Dmt-L-Lys-L-Phe-NH₂ |
| D-Arg-Dmt-D-Lys-NH₂ |
| D-Arg-Dmt-Lys-NH₂ |
| D-Arg-Dmt-Lys-Phe-Cys |
| D-Arg-L-Dmt-D-Lys-D-Phe-NH₂ |
| D-Arg-L-Dmt-D-Lys-L-Phe-NH₂ |
| D-Arg-L-Dmt-L-Lys-D-Phe-NH₂ |
| Dmt-Arg |
| Dmt-Lys |
| Dmt-Lys-Phe |
| Dmt-Phe-Arg-Lys |
| H-Arg-D-Dmt-Lys-Phe-NH₂ |
| H-Arg-Dmt-Lys-Phe-NH₂ |
| H-D-Arg-2,6-dichloro-L-tyrosine-L-Lys-L-Phe-NH₂ |
| H-D-Arg-2,6-dichlorotyrosine-Lys-Phe-NH₂ |
| H-D-Arg-2,6-difluoro-L-tyrosine-L-Lys-L-Phe-NH₂ |
| H-D-Arg-2,6-difluorotyrosine-Lys-Phe-NH₂ |
| H-D-Arg-2,6-dimethyl-L-phenylalanine-L-Lys-L-Phe-NH₂ |
| H-D-Arg-2,6-dimethylphenylalanine-Lys-Phe-NH₂ |
| H-D-Arg-4-methoxy-2,6-dimethyl-L-phenylalanine-L-Lys-L- Phe-NH2 |
| H-D-Arg-4-methoxy-2,6-dimethylphenylalanine-Lys-Phe-NH₂ |
| H-D-Arg-Dmt-Lys-2,6-dimethylphenylalanine-NH₂ |
| H-D-Arg-Dmt-Lys-3-hydroxyphenylalanine-NH₂ |
| H-D-Arg-Dmt-N6-acetyllysine-Phe-NH₂ |
| H-D-Arg-D-Phe-L-Lys-L-Phe-NH₂ |
| H-D-Arg-D-Trp-L-Lys-L-Phe-NH₂ |
| H-D-Arg-D-Tyr-L-Lys-L-Phe-NH₂ |
| H-D-Arg-L-Dmt-L-Lys-2,6-dimethyl-L-phenylalanine-NH₂ |
| H-D-Arg-L-Dmt-L-Lys-3-hydroxy-L-phenylalanine-NH₂ |
| H-D-Arg-L-Dmt-L-Lys-D-Dmt-NH₂ |
| H-D-Arg-L-Dmt-L-Lys-D-Trp-NH₂ |
| H-D-Arg-L-Dmt-L-Lys-D-Tyr-NH₂ |
| H-D-Arg-L-Dmt-L-Lys-L-Dmt-NH₂ |
| H-D-Arg-L-Dmt-L-Lys-L-Trp-NH₂ |
| H-D-Arg-L-Dmt-L-Lys-L-Tyr-NH₂ |
| H-D-Arg-L-Dmt-L-Phe-L-Lys-NH₂ |
| H-D-Arg-L-Dmt-N6-acetyl-L-lysine-L-Phe-NH₂ |
| H-D-Arg-L-Lys-L-Dmt-L-Phe-NH₂ |
| H-D-Arg-L-Lys-L-Phe-L-Dmt-NH₂ |
| H-D-Arg-L-Phe-L-Dmt-L-Lys-NH₂ |
| H-D-Arg-L-Phe-L-Lys-L-Dmt-NH₂ |
| H-D-Arg-L-Phe-L-Lys-L-Phe-NH₂ |
| H-D-Arg-L-Trp-L-Lys-L-Phe-NH₂ |
| H-D-Arg-L-Tyr-L-Lys-L-Phe-NH₂ |
| H-D-Arg-Phe-Lys-Dmt-NH₂ |
| H-D-Arg-Tyr-Lys-Phe-NH₂ |
| H-D-His-L-Dmt-L-Lys-L-Phe-NH₂ |
| H-D-Lys-L-Dmt-L-Lys-L-Phe-NH₂ |
| H-Dmt-D-Arg-Lys-Phe-NH₂ |
| H-Dmt-D-Arg-Phe-Lys-NH₂ |
| H-Dmt-Lys-D-Arg-Phe-NH₂ |
| H-Dmt-Lys-Phe-D-Arg-NH₂ |
| H-Dmt-Phe-D-Arg-Lys-NH₂ |
| H-Dmt-Phe-Lys-D-Arg-NH₂ |
| H-L-Dmt-D-Arg-L-Lys-L-Phe-NH₂ |
| H-L-Dmt-D-Arg-L-Phe-L-Lys-NH₂ |
| H-L-Dmt-L-Lys-D-Arg-L-Phe-NH₂ |
| H-L-Dmt-L-Lys-L-Phe-D-Arg-NH₂ |
| H-L-Dmt-L-Phe-D-Arg-L-Lys-NH₂ |
| H-L-Dmt-L-Phe-L-Lys-D-Arg-NH₂ |
| H-L-His-L-Dmt-L-Lys-L-Phe-NH₂ |
| H-L-Lys-D-Arg-L-Dmt-L-Phe-NH₂ |
| H-L-Lys-D-Arg-L-Phe-L-Dmt-NH₂ |
| H-L-Lys-L-Dmt-D-Arg-L-Phe-NH₂ |
| H-L-Lys-L-Dmt-L-Lys-L-Phe-NH₂ |
| H-L-Lys-L-Dmt-L-Phe-D-Arg-NH₂ |
| H-L-Lys-L-Phe-D-Arg-L-Dmt-NH₂ |
| H-L-Lys-L-Phe-L-Dmt-D-Arg-NH₂ |
| H-L-Phe-D-Arg-L-Dmt-L-Lys-NH₂ |
| H-L-Phe-D-Arg-L-Lys-L-Dmt-NH₂ |
| H-L-Phe-L-Dmt-D-Arg-L-Lys-NH₂ |
| H-L-Phe-L-Dmt-L-Lys-D-Arg-NH₂ |
| H-L-Phe-L-Lys-D-Arg-L-Dmt-NH₂ |
| H-L-Phe-L-Lys-L-Dmt-D-Arg-NH₂ |
| H-Lys-D-Arg-Dmt-Phe-NH₂ |
| H-Lys-D-Arg-Phe-Dmt-NH₂ |
| H-Lys-Dmt-D-Arg-Phe-NH₂ |
| H-Lys-Dmt-Phe-D-Arg-NH₂ |
| H-Lys-Phe-D-Arg-Dmt-NH₂ |
| H-Lys-Phe-Dmt-D-Arg-NH₂ |
| H-Phe-Arg-Phe-Lys-NH₂ |
| H-Phe-D-Arg-Dmt-Lys-NH₂ |
| H-Phe-D-Arg-Lys-Dmt-NH₂ |
| H-Phe-Dmt-D-Arg-Lys-NH₂ |
| H-Phe-Dmt-Lys-D-Arg-NH₂ |
| H-Phe-Lys-D-Arg-Dmt-NH₂ |
| H-Phe-Lys-Dmt-D-Arg-NH₂ |
| L-Arg-D-Dmt-D-Lys-D-Phe-NH₂ |
| L-Arg-D-Dmt-D-Lys-L-Phe-NH₂ |
| L-Arg-D-Dmt-L-Lys-D-Phe-NH₂ |
| L-Arg-D-Dmt-L-Lys-L-Phe-NH₂ |
| L-Arg-L-Dmt-D-Lys-D-Phe-NH₂ |
| L-Arg-L-Dmt-D-Lys-L-Phe-NH₂ |
| L-Arg-L-Dmt-L-Lys-D-Phe-NH₂ |
| L-Arg-L-Dmt-L-Lys-L-Phe-NH₂ |
| Lys-Dmt-Arg |
| Lys-Phe |
| Lys-Phe-Arg-Dmt |
| Lys-Trp-Arg |
| Phe-Arg-Dmt-Lys |
| Phe-Arg-Phe-Lys |
| Phe-Dmt-Arg-Lys |
| Phe-Lys-Dmt |
| Arg-Dmt-Lys-Phe-NH₂ |
| Phe-Dmt-Arg-Lys-NH₂ |
| Phe-Lys-Dmt-Arg-NH₂ |
| Dmt-Arg-Lys-Phe-NH₂ |
| Lys-Dmt-Arg-Phe-NH₂ |
| Phe-Dmt-Lys-Arg-NH₂ |
| Arg-Lys-Dmt-Phe-NH₂ |
| Arg-Dmt-Phe-Lys-NH₂ |
| D-Arg-Dmt-Lys-Phe-NH₂ |
| Dmt-D-Arg-Phe-Lys-NH₂ |
| H-Phe-D-Arg Phe-Lys-Cys-NH₂ |
| D-Arg-Dmt-Lys-Trp-NH₂ |
| D-Arg-Trp-Lys-Trp-NH₂ |
| H-D-Arg-Dmt-Lys-Phe(*N*Me)-NH₂ |
| H-D-Arg-Dmt-Lys(*N^{α}*Me)-Phe(*N*Me)-NH₂ |
| H-D-Arg(*N^{α}*Me)-Dmt(*N*Me)-Lys(*N^{α}*Me)-Phe(*N*Me)-NH₂ |
| D-Arg-2'6'Dmt-Lys-Phe-NH₂ |
| H-Phe-D-Arg-Phe-Lys-Cys-NH₂ |
| D-Arg-Dmt-Lys-Phe-Ser-Cys-NH₂ |
| D-Arg-Dmt-Lys-Phe-Gly-Cys-NH₂ |
| Gly-D-Phe-Lys-His-D-Arg-Tyr-NH₂ |
| D-Arg-Dmt-Lys-Phe-Met-NH₂ |
| D-Arg-Dmt-Lys-Phe-Lys-Trp-NH₂ |
| D-Arg-Dmt-Lys-Dmt-Lys-Trp-NH₂ |
| D-Arg-Dmt-Lys-Phe-Lys-Met-NH₂ |
| D-Arg-Dmt-Lys-Dmt-Lys-Met-NH₂ |
| H-D-Arg-Dmt-Lys-OH |
| H-D-Arg-Dmt-OH |
| H-D-Arg-Dmt-Lys-Phe-OH |

| **TABLE B** | | | | |
|---|---|---|---|---|
| **Amino Acid Position 1** | **Amino Acid Position 2** | **Amino Acid Position 3** | **Amino Acid Position 4** | **C-Terminal Modification** |
| Tyr | D-Arg | Phe | Orn | NH₂ |
| Tyr | D-Arg | Phe | Dab | NH₂ |
| Tyr | D-Arg | Phe | Dap | NH₂ |
| 2'6'Dmt | D-Arg | Phe | Lys-NH(CH₂)₂-NH-dns | NH₂ |
| 2'6'Dmt | D-Arg | Phe | Lys-NH(CH₂)₂-NH-atn | NH₂ |
| 2'6'Dmt | D-Arg | Phe | dnsLys | NH₂ |
| 2'6'Dmt | D-Cit | Phe | Ahp | NH₂ |
| 2'6'Dmt | D-Arg | Phe | Dab | NH₂ |
| 2'6'Dmt | D-Arg | Phe | Dap | NH₂ |
| 3'5'Dmt | D-Arg | Phe | Lys | NH₂ |
| 3'5'Dmt | D-Arg | Phe | Orn | NH₂ |
| 3'5'Dmt | D-Arg | Phe | Dab | NH₂ |
| 3'5'Dmt | D-Arg | Phe | Dap | NH₂ |
| Tyr | D-Arg | Tyr | Lys | NH₂ |
| Tyr | D-Arg | Tyr | Orn | NH₂ |
| Tyr | D-Arg | Tyr | Dab | NH₂ |
| Tyr | D-Arg | Tyr | Dap | NH₂ |
| 2'6'Dmt | D-Arg | Tyr | Lys | NH₂ |
| 2'6'Dmt | D-Arg | Tyr | Orn | NH₂ |
| 2'6'Dmt | D-Arg | Tyr | Dab | NH₂ |
| 2'6'Dmt | D-Arg | Tyr | Dap | NH₂ |
| 2'6'Dmt | D-Arg | 2'6'Dmt | Lys | NH₂ |
| 2'6'Dmt | D-Arg | 2'6'Dmt | Orn | NH₂ |
| 2'6'Dmt | D-Arg | 2'6'Dmt | Dab | NH₂ |
| 2'6'Dmt | D-Arg | 2'6'Dmt | Dap | NH₂ |
| 3'5'Dmt | D-Arg | 3'5'Dmt | Arg | NH₂ |
| 3'5'Dmt | D-Arg | 3'5'Dmt | Lys | NH₂ |
| 3'5'Dmt | D-Arg | 3'5'Dmt | Orn | NH₂ |
| 3'5'Dmt | D-Arg | 3'5'Dmt | Dab | NH₂ |
| Tyr | D-Lys | Phe | Dap | NH₂ |
| Tyr | D-Lys | Phe | Arg | NH₂ |
| Tyr | D-Lys | Phe | Lys | NH₂ |
| Tyr | D-Lys | Phe | Orn | NH₂ |
| 2'6'Dmt | D-Lys | Phe | Dab | NH₂ |
| 2'6'Dmt | D-Lys | Phe | Dap | NH₂ |
| 2'6'Dmt | D-Lys | Phe | Arg | NH₂ |
| 2'6'Dmt | D-Lys | Phe | Lys | NH₂ |
| 3'5'Dmt | D-Lys | Phe | Orn | NH₂ |
| 3'5'Dmt | D-Lys | Phe | Dab | NH₂ |
| 3'5'Dmt | D-Lys | Phe | Dap | NH₂ |
| 3'5'Dmt | D-Lys | Phe | Arg | NH₂ |
| Tyr | D-Lys | Tyr | Lys | NH₂ |
| Tyr | D-Lys | Tyr | Orn | NH₂ |
| Tyr | D-Lys | Tyr | Dab | NH₂ |
| Tyr | D-Lys | Tyr | Dap | NH₂ |
| 2'6'Dmt | D-Lys | Tyr | Lys | NH₂ |
| 2'6'Dmt | D-Lys | Tyr | Orn | NH₂ |
| 2'6'Dmt | D-Lys | Tyr | Dab | NH₂ |
| 2'6'Dmt | D-Lys | Tyr | Dap | NH₂ |
| 2'6'Dmt | D-Lys | 2'6'Dmt | Lys | NH₂ |
| 2'6'Dmt | D-Lys | 2'6'Dmt | Orn | NH₂ |
| 2'6'Dmt | D-Lys | 2'6'Dmt | Dab | NH₂ |
| 2'6'Dmt | D-Lys | 2'6'Dmt | Dap | NH₂ |
| 3'5'Dmt | D-Lys | 3'5'Dmt | Lys | NH₂ |
| 3'5'Dmt | D-Lys | 3'5'Dmt | Orn | NH₂ |
| 3'5'Dmt | D-Lys | 3'5'Dmt | Dab | NH₂ |
| 3'5'Dmt | D-Lys | 3'5'Dmt | Dap | NH₂ |
| Tyr | D-Lys | Phe | Arg | NH₂ |
| Tyr | D-Orn | Phe | Arg | NH₂ |
| Tyr | D-Dab | Phe | Arg | NH₂ |
| Tyr | D-Dap | Phe | Arg | NH₂ |
| 2'6'Dmt | D-Arg | Phe | Arg | NH₂ |
| 2'6'Dmt | D-Lys | Phe | Arg | NH₂ |
| 2'6'Dmt | D-Orn | Phe | Arg | NH₂ |
| 2'6'Dmt | D-Dab | Phe | Arg | NH₂ |
| 3'5'Dmt | D-Dap | Phe | Arg | NH₂ |
| 3'5'Dmt | D-Arg | Phe | Arg | NH₂ |
| 3'5'Dmt | D-Lys | Phe | Arg | NH₂ |
| 3'5'Dmt | D-Orn | Phe | Arg | NH₂ |
| Tyr | D-Lys | Tyr | Arg | NH₂ |
| Tyr | D-Orn | Tyr | Arg | NH₂ |
| Tyr | D-Dab | Tyr | Arg | NH₂ |
| Tyr | D-Dap | Tyr | Arg | NH₂ |
| 2'6'Dmt | D-Arg | 2'6'Dmt | Arg | NH₂ |
| 2'6'Dmt | D-Lys | 2'6'Dmt | Arg | NH₂ |
| 2'6'Dmt | D-Orn | 2'6'Dmt | Arg | NH₂ |
| 2'6'Dmt | D-Dab | 2'6'Dmt | Arg | NH₂ |
| 3'5'Dmt | D-Dap | 3'5'Dmt | Arg | NH₂ |
| 3'5'Dmt | D-Arg | 3'5'Dmt | Arg | NH₂ |
| 3'5'Dmt | D-Lys | 3'5'Dmt | Arg | NH₂ |
| 3'5'Dmt | D-Orn | 3'5'Dmt | Arg | NH₂ |
| Mmt | D-Arg | Phe | Lys | NH₂ |
| Mmt | D-Arg | Phe | Orn | NH₂ |
| Mmt | D-Arg | Phe | Dab | NH₂ |
| Mmt | D-Arg | Phe | Dap | NH₂ |
| Tmt | D-Arg | Phe | Lys | NH₂ |
| Tmt | D-Arg | Phe | Orn | NH₂ |
| Tmt | D-Arg | Phe | Dab | NH₂ |
| Tmt | D-Arg | Phe | Dap | NH₂ |
| Hmt | D-Arg | Phe | Lys | NH₂ |
| Hmt | D-Arg | Phe | Orn | NH₂ |
| Hmt | D-Arg | Phe | Dab | NH₂ |
| Hmt | D-Arg | Phe | Dap | NH₂ |
| Mmt | D-Lys | Phe | Lys | NH₂ |
| Mmt | D-Lys | Phe | Orn | NH₂ |
| Mmt | D-Lys | Phe | Dab | NH₂ |
| Mmt | D-Lys | Phe | Dap | NH₂ |
| Mmt | D-Lys | Phe | Arg | NH₂ |
| Tmt | D-Lys | Phe | Lys | NH₂ |
| Tmt | D-Lys | Phe | Orn | NH₂ |
| Tmt | D-Lys | Phe | Dab | NH₂ |
| Tmt | D-Lys | Phe | Dap | NH₂ |
| Tmt | D-Lys | Phe | Arg | NH₂ |
| Hmt | D-Lys | Phe | Lys | NH₂ |
| Hmt | D-Lys | Phe | Orn | NH₂ |
| Hmt | D-Lys | Phe | Dab | NH₂ |
| Hmt | D-Lys | Phe | Dap | NH₂ |
| Hmt | D-Lys | Phe | Arg | NH₂ |
| Mmt | D-Lys | Phe | Arg | NH₂ |
| Mmt | D-Orn | Phe | Arg | NH₂ |
| Mmt | D-Dab | Phe | Arg | NH₂ |
| Mmt | D-Dap | Phe | Arg | NH₂ |
| Mmt | D-Arg | Phe | Arg | NH₂ |
| Tmt | D-Lys | Phe | Arg | NH₂ |
| Tmt | D-Orn | Phe | Arg | NH₂ |
| Tmt | D-Dab | Phe | Arg | NH₂ |
| Tmt | D-Dap | Phe | Arg | NH₂ |
| Tmt | D-Arg | Phe | Arg | NH₂ |
| Hmt | D-Lys | Phe | Arg | NH₂ |
| Hmt | D-Orn | Phe | Arg | NH₂ |
| Hmt | D-Dab | Phe | Arg | NH₂ |
| Hmt | D-Dap | Phe | Arg | NH₂ |
| Hmt | D-Arg | Phe | Arg | NH₂ |
| Trp | D-Arg | Phe | Lys | NH₂ |

2'-methyltyrosine (Mmt); Dimethyltyrosine (Dmt); 2',6'-dimethyltyrosine (2'6'-Dmt); 3',5'-dimethyltyrosine (3'5'Dmt); N,2',6'-trimethyltyrosine (Tmt); 2'-hydroxy-6'-methyltyrosine (Hmt); 2'-methylphenylalanine (Mmp); dimethylphenylalanine (Dmp) 2',6'-dimethylphenylalanine (2',6'-Dmp); N,2',6'-trimethylphenylalanine (Tmp); 2'-hydroxy-6'-methylphenylalanine (Hmp); cyclohexylalanine (Cha); diaminobutyric (Dab); diaminopropionic acid (Dap); β-dansyl-L-α,β -diaminopropionic acid (dnsDap); β-anthraniloyl-L-α,β-diaminopropionic acid (atnDap); biotin (bio); norleucine (Nle); 2-aminohepantoic acid (Ahp); β-(6'-dimethylamino-2'-naphthoyl)alanine (Ald); Sarcosine (Sar)

In another embodiment, the peptide is defined by Formula II: 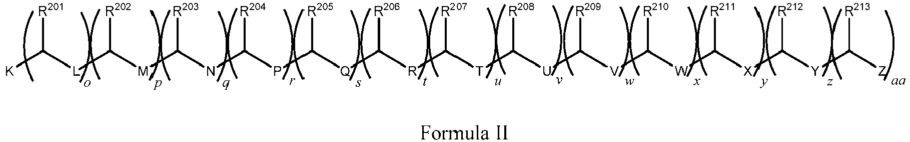 wherein:
one of K and Z is 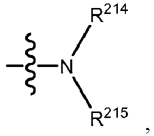
and the other of K and Z is 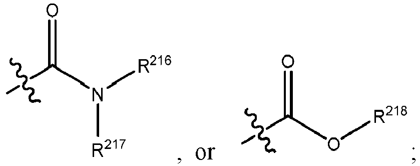
L, M, N, P, Q, R, T, U, V, W, X, and Y are each 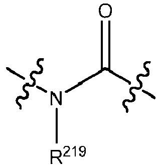 or L, M, N, P, Q, R, T, U, V, W, X, and Y are each 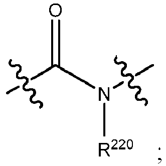 with the proviso that when
   *aa* is 0 and Z is not a terminal group, the terminal group is one of L, M, N, P, Q, R, T, U, V, W, X, or Y, such that one of K and the terminal group is 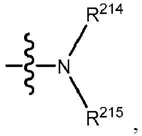
   and the other of K and the terminal group is selected from 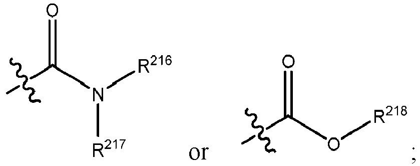
R²⁰¹ is 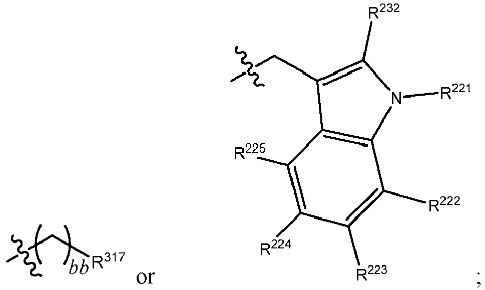
R²⁰² is 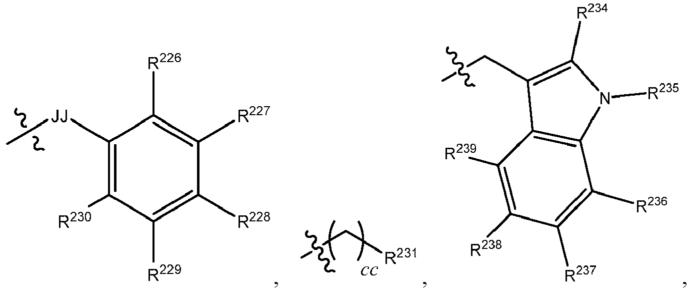 or 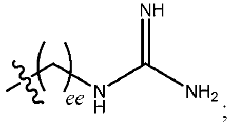
R²⁰³ is 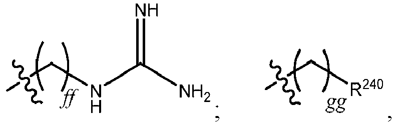 or hydrogen;
R²⁰⁴ is 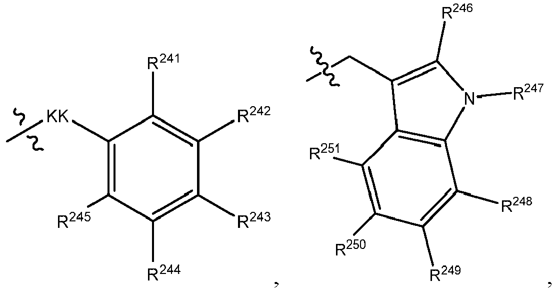 or 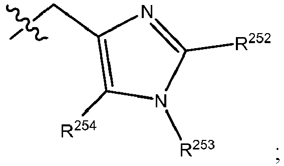
R²⁰⁵ is 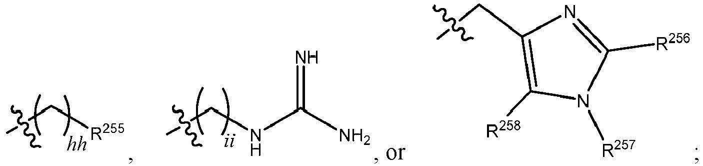
R²⁰⁶ is 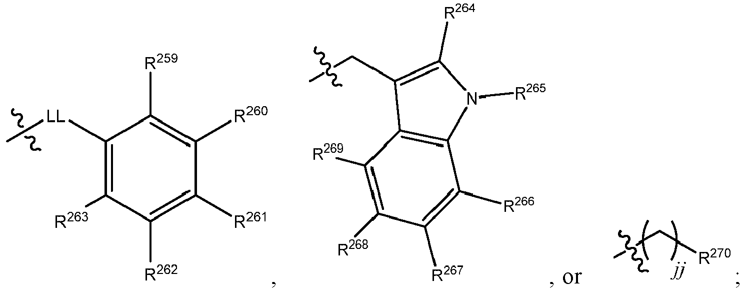
R²⁰⁷ is 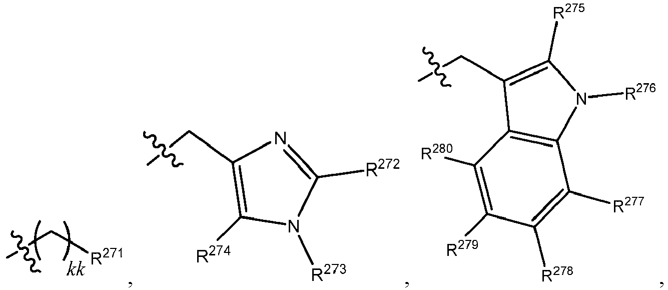 or hydrogen;
R²⁰⁸ is 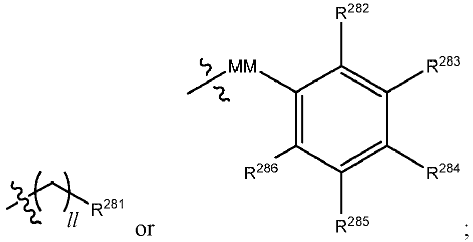
R²⁰⁹ is 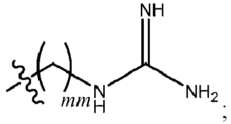
R²¹⁰ is 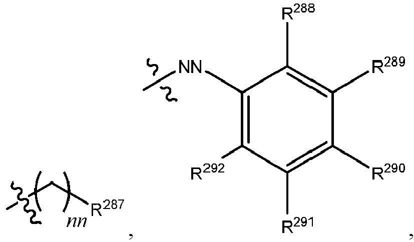 or hydrogen;
R²¹¹ is 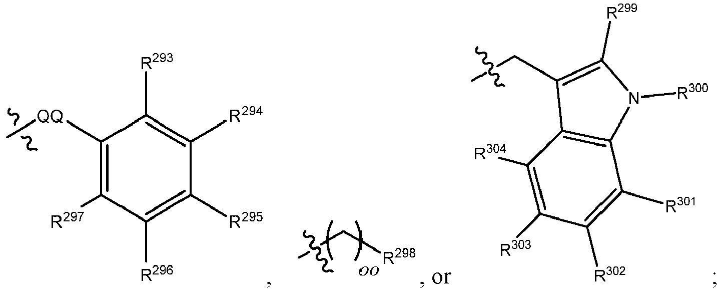
R²¹² is 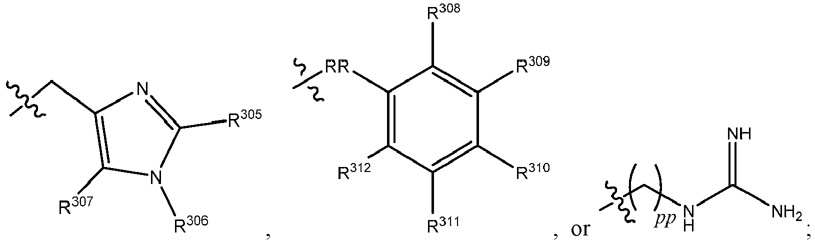
R²¹³ is 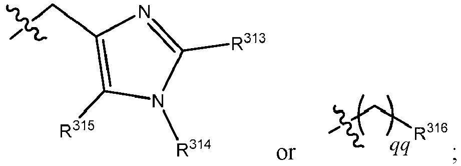 wherein
   R²¹⁴, R²¹⁵, R²¹⁶, R²¹⁷, and R²¹⁸ are each independently a hydrogen or substituted or unsubstituted C₁-C₆ alkyl, C₂-C₆ alkenyl, C₂-C₆ alkynyl, saturated or unsaturated cycloalkyl, cycloalkylalkyl, aryl, aralkyl, 5- or 6- membered saturated or unsaturated heterocylyl, heteroaryl, or amino protecting group; or R²¹⁴ and R²¹⁵ together form a 3, 4, 5, 6, 7, or 8 membered substituted or unsubstituted heterocycyl ring;
   R²¹⁹ and R²²⁰ are, at each occurrence, independently a hydrogen or substituted or unsubstituted C₁-C₆ alkyl group;
   R²²², R²²³, R²²⁴, R²²⁵, R²²⁶, R²²⁷, R²²⁸, R²²⁹, R²³⁰, R²³², R²³⁴, R²³⁶, R²³⁷, R²³⁸, R²³⁹, R²⁴¹, R²⁴², R²⁴³, R²⁴⁴, R²⁴⁵, R²⁴⁶, R²⁴⁸, R²⁴⁹, R²⁵⁰, R²⁵¹, R²⁵², R²⁵⁴, R²⁵⁶, R²⁵⁸, R²⁵⁹, R²⁶⁰, R²⁶¹, R²⁶², R²⁶³, R²⁶⁴, R²⁶⁶, R²⁶⁷, R²⁶⁸, R²⁶⁹, R²⁷², R²⁷⁴, R²⁷⁵, R²⁷⁷, R²⁷⁸, R²⁷⁹, R²⁸⁰, R²⁸², R²⁸³, R²⁸⁴, R²⁸⁵, R²⁸⁶, R²⁸⁸, R²⁸⁹, R²⁹⁰, R²⁹¹, R²⁹², R²⁹³, R²⁹⁴, R²⁹⁵, R²⁹⁶, R²⁹⁷, R²⁹⁹, R³⁰¹, R³⁰², R³⁰³, R³⁰⁴, R³⁰⁵, R³⁰⁷, R³⁰⁸, R³⁰⁹, R³¹⁰, R³¹¹, R³¹², R³¹³, and R³¹⁵ are each independently a hydrogen, amino, amido, -NO₂, -CN, -OR^{c}, - SR^{c}, -NR^{c}R^{c}, -F, -Cl, -Br, -I, or a substituted or unsubstituted C₁-C₆ alkyl, C₁-C₆ alkoxy, -C(O)-alkyl, -C(O)-aryl, -C(O)-aralkyl, -C(O)₂R^{c}, C₁-C₄ alkylamino, C₁-C₄ dialkylamino, or perhaloalkyl group;
   R²²¹, R²³⁵, R²⁴⁷, R²⁵³, R²⁵⁷, R²⁶⁵, R²⁷³, R²⁷⁶, R³⁰⁰, R³⁰⁶, and R³¹⁴ are each independently a hydrogen or substituted or unsubstituted C₁-C₆ alkyl group;
   R²³¹, R²⁴⁰, R²⁵⁵, R²⁷⁰, R²⁷¹, R²⁸¹, R²⁸⁷, R²⁹⁸, R³¹⁶, and R³¹⁷ are each independently a hydrogen, -OR^{c}, -SR^{c}, -NR^{c}R^{c}, -NR^{c}R^{d}, -CO₂R^{c}, -(CO)NR^{c}R^{c}, -NR^{c}(CO)R^{c}, -NR^{c}C(NH)NH₂, -NR^{c}-dansyl, or a substituted or unsubstituted alkyl, aryl, or aralkyl group;
   JJ, KK, LL, MM, NN, QQ, and RR are each independently absent, -NH(CO)-, or -CH₂-;
   R^{c} at each occurrence is independently a hydrogen or a substituted or unsubstituted C₁-C₆ alkyl group;
   R^{d} at each occurrence is independently a C₁-C₆ alkylene-NR^{c}-dansyl or C₁-C₆ alkylene-NR^{c}-anthraniloyl group;
   *o, p, q, r, s, t, u, v*, *w, x, y, z,* and *aa* are each independently 0 or 1,
      with the proviso that *o* + *p* + *q* + *r* + *s + t* + *u* + *v* + *w* + *x* + *y* + *z* + *aa* equals 6, 7, 8, 9, 10, or 11;
   *cc* is 0, 1, 2, 3, 4, or 5; and
   *bb, cc, ee, ff, gg, hh, ii, jj, kk, ll, mm, nn, oo, pp,* and *qq* are each independently 1, 2, 3, 4, or 5.

In some embodiments of peptides of Formula II,
R²¹⁴, R²¹⁵, R²¹⁶, R²¹⁷, and R²¹⁸ are each independently a hydrogen or substituted or unsubstituted C₁-C₆ alkyl group;
R²¹⁹ and R²²⁰ are, at each occurrence, independently a hydrogen or methyl group;
R²²², R²²³, R²²⁴, R²²⁵, R²²⁶, R²²⁷, R²²⁸, R²²⁹, R²³⁰, R²³², R²³⁴, R²³⁶, R²³⁷, R²³⁸, R²³⁹, R²⁴¹, R²⁴², R²⁴³, R²⁴⁴, R²⁴⁵, R²⁴⁶, R²⁴⁸, R²⁴⁹, R²⁵⁰, R²⁵¹, R²⁵², R²⁵⁴, R²⁵⁶, R²⁵⁸, R²⁵⁹, R²⁶⁰, R²⁶¹, R²⁶², R²⁶³, R²⁶⁴, R²⁶⁶, R²⁶⁷, R²⁶⁸, R²⁶⁹, R²⁷², R²⁷⁴, R²⁷⁵, R²⁷⁷, R²⁷⁸, R²⁷⁹, R²⁸⁰, R²⁸², R²⁸³, R²⁸⁴, R²⁸⁵, R²⁸⁶, R²⁸⁸, R²⁸⁹, R²⁹⁰, R²⁹¹, R²⁹², R²⁹³, R²⁹⁴, R²⁹⁵, R²⁹⁶, R²⁹⁷, R²⁹⁹, R³⁰¹, R³⁰², R³⁰³, R³⁰⁴, R³⁰⁵, R³⁰⁷, R³⁰⁸, R³⁰⁹, R³¹⁰, R³¹¹, R³¹², R³¹³, and R³¹⁵ are each independently a hydrogen, methyl, or -OR^{c} group;
R²²¹, R²³⁵, R²⁴⁷, R²⁵³, R²⁵⁷, R²⁶⁵, R²⁷³, R²⁷⁶, R³⁰⁰, R³⁰⁶, and R³¹⁴ are each independently a hydrogen or substituted or unsubstituted C₁-C₆ alkyl group;
R²³¹ is -(CO)NR^{c}R^{c}, -OR^{c}, or a C₁-C₆ alkyl group, optionally substituted with a hydroxyl or methyl group;
R²⁴⁰ and R²⁵⁵ are each independently -CO₂R^{c} or -NR^{c}R^{c};
R²⁷⁰ and R²⁷¹ are each independently -CO₂R^{c};
R²⁸¹ is -SR^{c} or -NR^{c}R^{c};
R²⁸⁷ -(CO)NR^{c}R^{c} or -OR^{c};
R²⁹⁸ -NR^{c}R^{c} , -CO₂R^{c} , or -SR^{c};
R³¹⁶ is -NR^{c}R^{c};
R³¹⁷ is hydrogen or -NR^{c}R^{c};
JJ, KK, LL, MM, NN, QQ, and RR are each independently absent or -CH₂-;
R^{c} at each occurrence is independently a hydrogen or a substituted or unsubstituted C₁-C₆ alkyl group;
R^{d} at each occurrence is independently a C₁-C₆ alkylene-NR^{c}-dansyl or C₁-C₆ alkylene-NR^{c}-anthraniloyl group;
*o, p, q, r, s, t, u, v, w, x, y, z,* and *aa* are each independently 0 or 1,
   with the proviso that *o* + *p* + *q* + *r* + *s* + *t* + *u* + *v* + *w* + *x* + *y* + *z* + *aa* equals 6, 7, 8, 9, 10, or 11;
*cc* is 0, 1, 2, 3, 4, or 5; and
*bb, cc, dd, ee, ff, gg, hh, ii, jj, kk, ll, mm, nn, oo, pp,* and *qq* are each independently 1, 2, 3, 4, or 5.

In some embodiments of peptides of Formula II,
R²²¹, R²²², R²²³, R²²⁴, R²²⁵, R²²⁶, R²²⁷, R²²⁸, R²²⁹, R²³⁰, R²³², R²³⁴, R²³⁵, R²³⁶, R²³⁷, R²³⁸, R²³⁹, R²⁴², R²⁴⁴, R²⁴⁶, R²⁴⁷, R²⁴⁸, R²⁴⁹, R²⁵⁰, R²⁵¹, R²⁵², R²⁵³, R²⁵⁴, R²⁵⁶, R²⁵⁷, R²⁵⁸, R²⁵⁹, R²⁶⁰, R²⁶², R²⁶³, R²⁶⁴, R²⁶⁵, R²⁶⁶, R²⁶⁷, R²⁶⁸, R²⁶⁹, R²⁷², R²⁷³, R²⁷⁴, R²⁷⁵, R²⁷⁶, R²⁷⁷, R²⁷⁸, R²⁷⁹, R²⁸⁰, R²⁸², R²⁸³, R²⁸⁵, R²⁸⁶, R²⁸⁸, R²⁸⁹, R²⁹¹, R²⁹², R²⁹³, R²⁹⁴, R²⁹⁶, R²⁹⁷, R²⁹⁹, R³⁰⁰, R³⁰¹, R³⁰², R³⁰³, R³⁰⁴, R³⁰⁵, R³⁰⁶, R³⁰⁷, R³⁰⁸, R³⁰⁹, R³¹¹, R³¹², R³¹³, R³¹⁴, and R³¹⁵ are each hydrogen;
R²⁴¹ and R²⁴⁵ are each independently a hydrogen or methyl group;
R²⁴³, R²⁶¹, R²⁸⁴, R²⁹⁰, R²⁹⁵, R³¹⁰ are each independently a hydrogen or OH;
R²³¹ is -(CO)NH₂, an ethyl group substituted with a hydroxyl group, or an isopropyl group;
R²⁴⁰ and R²⁵⁵ are each independently -CO₂H or -NH₂;
R²⁷⁰ and R²⁷¹ are each independently -CO₂H;
R²⁸¹ is -SH or -NH₂;
R²⁸⁷ is -(CO)NH₂ or -OH;
R²⁹⁸ is -NH₂, -CO₂H, or -SH;
R³¹⁶ is -NH₂;
R³¹⁷ is hydrogen or -NH₂;
JJ, KK, LL, MM, NN, QQ, and RR are each independently -CH₂-;
*o, p, q, r, s, t, u, v, w, x, y, z,* and *aa* are each independently 0 or 1,
   with the proviso that *o* + *p* + *q* + *r* + *s* + *t* + *u* + *v* + *w* + *x* + *y* + *z* + *aa* equals 6, 7, 8, 9, 10, or 11;
*cc* is 0, 1, 2, 3, 4, or 5; and
*bb, cc, dd, ee, ff, gg, hh, ii, jj, kk, ll, mm, nn, oo, pp,* and *qq* are each independently 1, 2, 3, 4, or 5.

In certain embodiments of Formula II,
K is 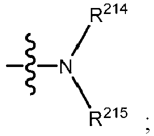
Z is 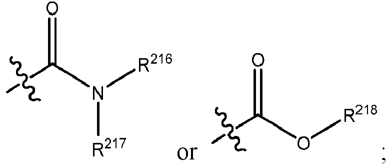
L, M, N, P, Q, R, T, U, V, W, X, and Y are each independently 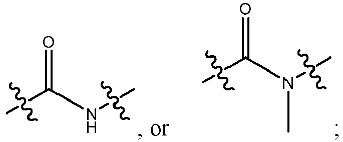 with the proviso that when
   *aa* is 0 and Z is not a terminal group, the terminal group is one of L, M, N, P, Q, R, T, U, V, W, X, or Y, such that one of L, M, N, P, Q, R, T, U, V, W, X, or Y, is

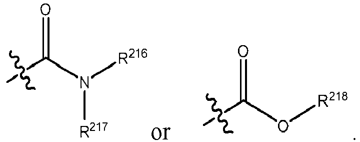

In another embodiment of Formula II,
K is 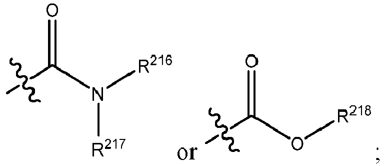
Z is 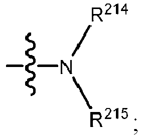
L, M, N, P, Q, R, T, U, V, W, X, and Y are each independently 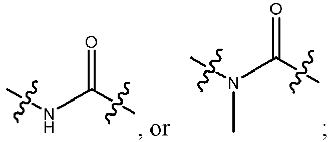 with the proviso that when
   *aa* is 0 and Z is not a terminal group, the terminal group is one of L, M, N, P, Q, R, T, U, V, W, X, or Y, such that one of L, M, N, P, Q, R, T, U, V, W, X, or Y, is

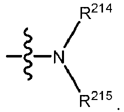

In some embodiments, the peptide of Formula II is selected from the peptides shown in Table C.

| **TABLE C** |
|---|
| D-Arg-Dmt-Lys-Phe-Glu-Cys-Gly-NH₂ |
| Phe-D-Arg-Phe-Lys-Glu-Cys-Gly-NH₂ |
| Phe-D-Arg-Dmt-Lys-Glu-Cys-Gly-NH₂ |
| Ala-D-Phe-D-Arg-Tyr-Lys-D-Trp-His-D-Tyr-Gly-Phe |
| Asp-D-Trp-Lys-Tyr-D-His-Phe-Arg-D-Gly-Lys-NH₂ |
| D-His-Glu-Lys-Tyr-D-Phe-Arg |
| D-His-Lys-Tyr-D-Phe-Glu-D-Asp-D-Asp-D-His-D-Lys-Arg-Trp- NH2 |
| Lys-D-Gln-Tyr-Arg-D-Phe-Trp-NH₂ |
| Lys-Trp-D-Tyr-Arg-Asn-Phe-Tyr-D-His-NH₂ |
| Phe-D-Arg-Lys-Trp-Tyr-D-Arg-His |
| Thr-Gly-Tyr-Arg-D-His-Phe-Trp-D-His-Lys |
| Tip-Lvs-Phe-D-Asp-Arg-Tyr-D-His-Lys |
| Val-D-Lys-His-Tyr-D-Phe-Ser-Tyr-Arg-NH₂ |
| Gly-D-Phe-Lys-Tyr-His-D-Arg-Tyr-NH₂ |
| Asp-D-Trp-Lys-Tyr-D-His-Phe-Arg-D-Gly-Lys-NH₂ |
| D-His-Lys-Tyr-D-Phe-Glu-D-Asp-D-His-D-Lys-Arg-Trp-NH₂. |
| H-Phe-D-Arg-Phe-Lys-Glu-Cys-Gly-NH₂ |
| Phe-Arg-Phe-Lys-Glu-Cys-Gly |
| H-D-Arg-Dmt-Lys-Phe-Sar-Gly-Cys-NH₂ |

In another embodiment the peptide is defined by Formula III: 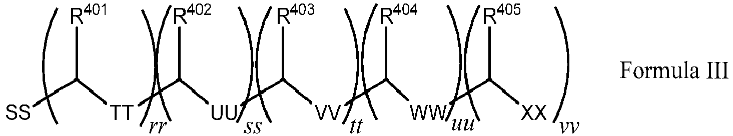 wherein:
one of SS and XX is 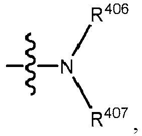
and the other is 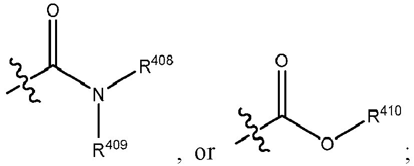
TT, UU, VV, and WW are each 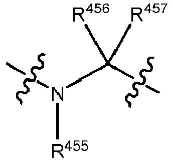 or TT, UU, VV, and WW are each 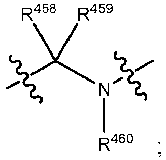
with the proviso when *vv* is 0 and *uu* is 1, one of SS and WW is 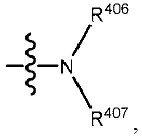
and the other of SS and WW is 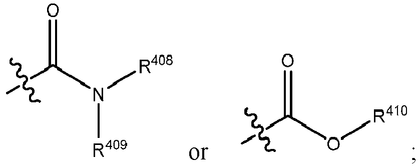
R⁴⁰¹ is 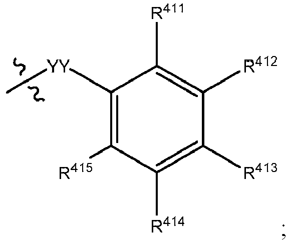
R⁴⁰² is 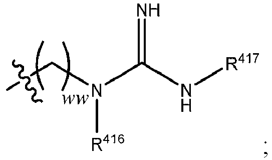
R⁴⁰³ is 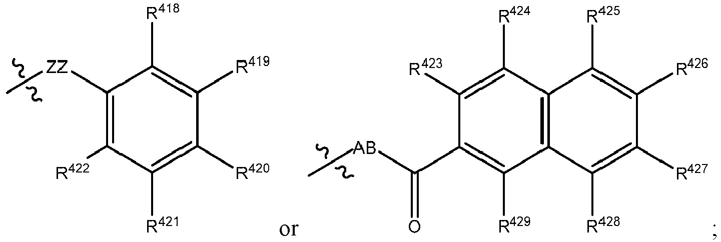
R⁴⁰⁴ is 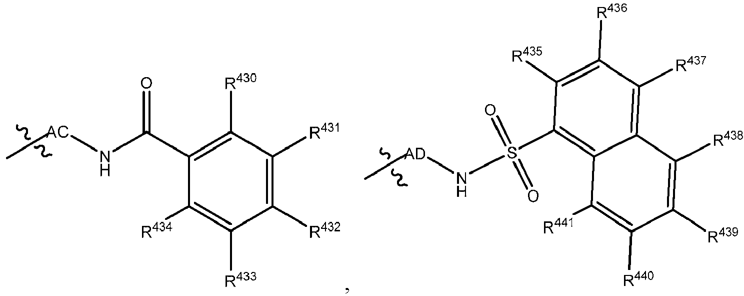 or, 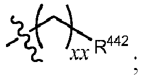
R⁴⁰⁵ is 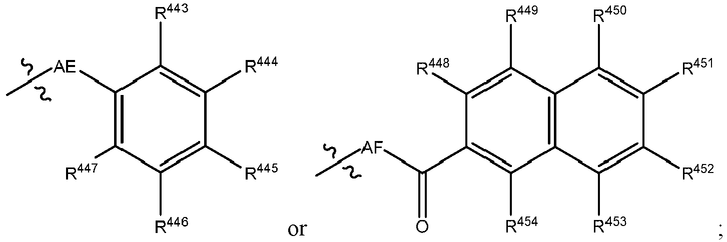 wherein
   R⁴⁰⁶, R⁴⁰⁷, R⁴⁰⁸ , R⁴⁰⁹, and R⁴¹⁰ are each independently a hydrogen or substituted or unsubstituted C₁-C₆ alkyl, C₂-C₆ alkenyl, C₂-C₆ alkynyl, saturated or unsaturated cycloalkyl, cycloalkylalkyl, aryl, aralkyl, 5- or 6- membered saturated or unsaturated heterocylyl, heterobicycyl, heteroaryl, or amino protecting group; or R⁴⁰⁶ and R⁴⁰⁷ together form a 3-, 4-, 5-, 6-, 7-, or 8-member substituted or unsubstituted heterocycyl ring;
   R⁴⁵⁵ and R⁴⁶⁰ are at each occurrence independently a hydrogen, - C(O)R^{e}, or an unsubstituted C₁-C₆ alkyl group;
   R⁴⁵⁶ and R⁴⁵⁷ are each independently a hydrogen or substituted or unsubstituted C₁-C₆ alkyl group; or together R⁴⁵⁶ and R⁴⁵⁷ are C=O;
   R⁴⁵⁸ and R⁴⁵⁹ are each independently a hydrogen or substituted or unsubstituted C₁-C₆ alkyl group; or together R⁴⁵⁸ and R⁴⁵⁹ are C=O;
   R⁴¹¹, R⁴¹², R⁴¹³, R⁴¹⁴, R⁴¹⁵, R⁴¹⁸, R⁴¹⁹, R⁴²⁰, R⁴²¹, R⁴²², R⁴²³, R⁴²⁴, R⁴²⁵, R⁴²⁶, R⁴²⁷, R⁴²⁸, R⁴²⁹, R⁴³⁰, R⁴³¹, R⁴³², R⁴³³, R⁴³⁴, R⁴³⁵, R⁴³⁶, R⁴³⁷, R⁴³⁸, R⁴³⁹, R⁴⁴⁰, R⁴⁴¹, R⁴⁴³, R⁴⁴⁴, R⁴⁴⁵, R⁴⁴⁶, R⁴⁴⁷, R⁴⁴⁸, R⁴⁴⁹, R⁴⁵⁰, R⁴⁵¹, R⁴⁵², R⁴⁵³, and R⁴⁵⁴ are each independently a hydrogen, deuterium, amino, amido, -NO₂, -CN, -OR^{e}, -SR^{e}, - NR^{e}R^{e}, -F, -Cl, -Br, -I, or a substituted or unsubstituted C₁-C₆ alkyl, C₁-C₆ alkoxy, -C(O)-alkyl, -C(O)-aryl, -C(O)-aralkyl, -C(O)₂R^{e}, C₁-C₄ alkylamino, C₁-C₄ dialkylamino, or perhaloalkyl group;
   R⁴¹⁶ and R⁴¹⁷ are each independently a hydrogen, -C(O)R^{e}, or a substituted or unsubstituted C₁-C₆ alkyl;
   R⁴⁴² is a hydrogen, -OR^{e}, -SR^{e}, -NR^{e}R^{e}, -NR^{e}R^{f}, -CO₂R^{e}, -C(O)NR^{e}R^{e}, -NR^{e}C(O)R^{e}, -NR^{e}C(NH)NH₂, -NR^{e}-dansyl, or a substituted or unsubstituted alkyl, aryl, or aralkyl group;
   YY, ZZ, and AE are each independently absent, -NH(CO)-, or -CH₂-;
   AB, AC, AD, and AF are each independently absent or C₁-C₆ alkylene group;
   R^{e} at each occurrence is independently a hydrogen or a substituted or unsubstituted C₁-C₆ alkyl group;
   R^{f} at each occurrence is independently a C₁-C₆ alkylene-NR^{e}-dansyl or C₁-C₆ alkylene-NR^{e}-anthraniloyl group;
   *rr, ss,* and vv are each independently 0 or 1; *tt* and *uu* are each 1 with the proviso that *rr* + *ss* + *tt* + *uu* + *vv* equals 4 or 5; and
   *ww* and *xx* are each independently 1, 2, 3, 4, or 5.

In some embodiments of peptides of Formula III,
R⁴⁰⁶ is a hydrogen, substituted or unsubstituted C₁-C₆ alkyl group, 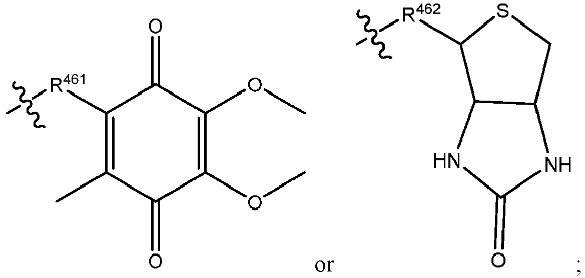 whe
   rein R⁴⁶¹ is a -C₁-C₁₀ alkylene-CO₂- or -CO₂-C₁-C₁₀ alkylene-CO₂-; and
   R⁴⁶² is C₁-C₁₀ alkylene or C₁-C₁₀ alkylene-CO₂-;
R⁴⁰⁷, R⁴⁰⁸, R⁴⁰⁹, and R⁴¹⁰ are each independently a hydrogen or substituted or unsubstituted C₁-C₆ alkyl group;
R⁴⁵⁵ and R⁴⁶⁰ are each independently a hydrogen, -C(O)-C₁-C₆ alkyl, or methyl group;
R⁴⁵⁶ and R⁴⁵⁷ are each a hydrogen or together R⁴⁵⁶ and R⁴⁵⁷ are C=O;
R⁴⁵⁸ and R⁴⁵⁹ are each a hydrogen or together R⁴⁵⁸ and R⁴⁵⁹ are C=O;
R⁴¹⁶ and R⁴¹⁷ are each independently a hydrogen or -C(O)R^{e};
R⁴¹¹, R⁴¹², R⁴¹³, R⁴¹⁴, R⁴¹⁵, R⁴¹⁸, R⁴¹⁹, R⁴²⁰, R⁴²¹, R⁴²², R⁴⁴³, R⁴⁴⁴, R⁴⁴⁵, R⁴⁴⁶, and R⁴⁴⁷ are each independently a hydrogen, deuterium, methyl, or -OR^{e} group;
R⁴²³, R⁴²⁴, R⁴²⁵, R⁴²⁶, R⁴²⁷, R⁴²⁸, R⁴²⁹, R⁴³⁰, R⁴³¹, R⁴³², R⁴³³, R⁴³⁴, R⁴³⁵, R⁴³⁶, R⁴³⁷, R⁴³⁸, R⁴³⁹, R⁴⁴⁰, R⁴⁴¹, R⁴⁴⁸, R⁴⁴⁹, R⁴⁵⁰, R⁴⁵¹, R⁴⁵², R⁴⁵³, and R⁴⁵⁴ are each independently a hydrogen, NR^{e}R^{e}, or substituted or unsubstituted C₁-C₆ alkyl group;
R⁴⁴² is a -NR^{e}R^{e};
YY, ZZ, and AE are each independently absent or -CH₂-;
AB, AC, AD, and AF are each independently absent or C₁-C₄ alkylene group;
R^{e} at each occurrence is independently a hydrogen or a substituted or unsubstituted C₁-C₆ alkyl group;
*rr, ss,* and vv are each independently 0 or 1; *tt* and *uu* are each 1
   with the proviso that *rr* + *ss* + *tt* + *uu* + *vv* equals 4 or 5; and
*ww* and *xx* are each independently 1, 2, 3, 4, or 5.

In some embodiments of peptides of Formula III,
R⁴⁰⁶ is 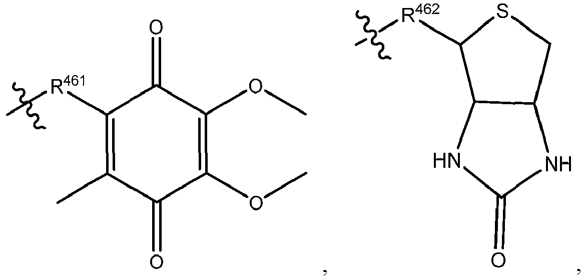 hydrogen, or methyl,
   wherein R⁴⁶¹ is a -(CH₂)₃-CO₂-, -(CH₂)₉-CO₂-, or -CO₂-(CH₂)₂-CO2- and R⁴⁶² is - (CH₂)₄-CO₂-;
   R⁴⁰⁷, R⁴⁰⁸, R⁴⁰⁹, and R⁴¹⁰ are each a hydrogen or methyl group;
   R⁴⁵⁵ and R⁴⁶⁰ are each independently a hydrogen, -C(O)CH₃, or methyl group;
   R⁴⁵⁶ and R⁴⁵⁷ are each a hydrogen or together R⁴⁵⁶ and R⁴⁵⁷ are C=O;
   R⁴⁵⁸ and R⁴⁵⁹ are each a hydrogen or together R⁴⁵⁸ and R⁴⁵⁹ are C=O;
   R⁴¹⁶ and R⁴¹⁷ are each independently a hydrogen or -C(O)CH₃;
   R⁴²⁶, R⁴³⁸, and R⁴⁵¹ are each -N(CH₃)₂;
   R⁴³⁴ and R⁴⁴² are each -NH₂;
   R⁴²³, R⁴²⁴, R⁴²⁵, R⁴²⁷, R⁴²⁸, R⁴²⁹, R⁴³⁰, R⁴³¹, R⁴³², R⁴³³, R⁴³⁵, R⁴³⁶, R⁴³⁷, R⁴³⁹, R⁴⁴⁰, R⁴⁴¹, R⁴⁴³, R⁴⁴⁴, R⁴⁴⁵, R⁴⁴⁶, R⁴⁴⁷, R⁴⁴⁸, R⁴⁴⁹, R⁴⁵⁰, R⁴⁵², R⁴⁵³, and R⁴⁵⁴ are each hydrogen;
   R⁴¹², R⁴¹⁴, R⁴¹⁹, and R⁴²¹ are each independently hydrogen or deuterium;
   R⁴¹¹, R⁴¹⁵, R⁴¹⁸, and R⁴²² are each independently hydrogen, deuterium, or methyl;
   R⁴¹³ and R⁴²⁰ are each independently hydrogen, deuterium, or OR^{e};
   YY, ZZ, and AE are each independently -CH₂-;
   AB, AC, AD, and AF are each -CH₂- or a butylene group;
   R^{e} at each occurrence is independently a hydrogen or a substituted or unsubstituted C₁-C₆ alkyl group;
   *rr, ss,* and *vv* are each independently 0 or 1; *tt* and *uu* are each 1
      with the proviso that *rr* + *ss* + *tt* + *uu* + *vv* equals 4 or 5; and
   ww and xx are each independently 3 or 4.

In certain embodiments of Formula III,
SS is 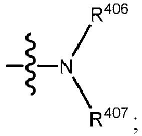
XX is 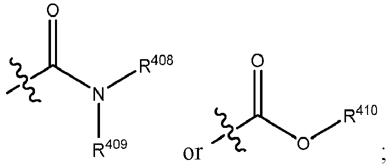
TT, UU, VV, and WW are each independently 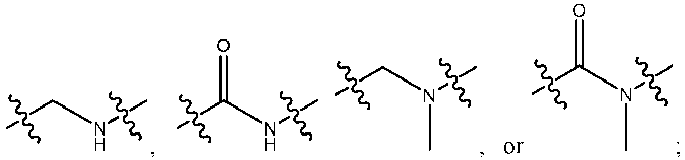 with the proviso when *vv* is 0 and *uu* is 1, WW is

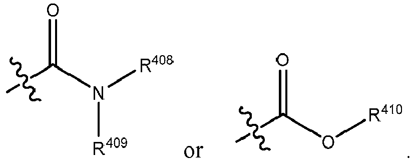

In some embodiments, the peptide of Formula III is selected from the peptides shown in Table D.

| **TABLE D** |
|---|
| 6-Butyric acid CoQ0-Phe-D-Arg-Phe-Lys-NH₂ |
| 6-Decanoic acid CoQ0-Phe-D-Arg-Phe-Lys-NH₂ |
| H-D-N2-acetylarginine-Dmt-Lys-Phe-NH₂ |
| H-D-N8-acetylarginine-Dmt-Lys-Phe-NH₂ |
| H-N2-acetyl-D-arginine-L-Dmt-L-Lys-L-Phe-NH₂ |
| H-N7-acetyl-D-arginine-Dmt-Lys-Phe-NH₂ |
| H-Phe(d5)-D-Arg-Phe(d5)-Lys-NH₂ |
| Succinic monoester CoQ0-Phe-D-Arg-Phe-Lys-HN₂ |
| Dmt-D-Arg-Phe-(atn)Dap-NH₂ |
| Dmt-D-Arg-Phe-(dns)Dap-NH₂ |
| Dmt-D-Arg-Ald-Lys-NH₂ |
| Dmt-D-Arg-Phe-Lys-Ald-NH₂ |
| Bio-2'6'Dmt-D-Arg-Phe-Lys-NH₂ |
| 2'6'Dmt-D-Arg-Phe-dnsDap-NH₂ |
| 2'6'Dmt-D-Arg-Phe-atnDap-NH₂ |
| H-D-Arg-Ψ[CH₂-NH]Dmt-Lys-Phe-NH₂ |
| H-D-Arg-Dmt-Ψ[CH₂-NH]Lys-Phe-NH₂ |
| H-D-Arg-Dmt-LysΨ[CH₂-NH]Phe-NH₂ |
| H-D-Arg-Dmt-Ψ[CH₂-NH]Lys-Ψ[CH₂-NH]Phe-NH₂ |

In some embodiments, the peptide is selected from the peptides shown in Table E.

| **TABLE E** |
|---|
| Arg-D-Leu-D-Tyr-Phe-Lys-Glu-D-Lys-Arg-D-Trp-Lys-D-Phe- Tyr-D-Arg-Gly |
| Asp-Arg-D-Phe-Cys-Phe-D-Arg-D-Lys-Tyr-Arg-D-Tyr-Trp-D- His-Tyr-D-Phe-Lys-Phe |
| D-Glu-Asp-Lys-D-Arg-D-His-Phe-Phe-D-Val-Tyr-Arg-Tyr-D- Tyr-Arg-His-Phe-NH2 |
| Glu-Arg-D-Lys-Tyr-D-Val-Phe-D-His-Trp-Arg-D-Gly-Tyr-Arg- D-Met-NH2 |
| Gly-Ala-Lys-Phe-D-Lys-Glu-Arg-Tyr-His-D-Arg-D-Arg-Asp- Tyr-Trp-D-His-Trp-His-D-Lys-Asp |
| His-Tyr-D-Arg-Trp-Lys-Phe-D-Asp-Ala-Arg-Cys-D-Tyr-His- Phe-D-Lys-Tyr-His-Ser-NH2 |
| Phe-Phe-D-Tyr-Arg-Glu-Asp-D-Lys-Arg-D-Arg-His-Phe-NH₂ |
| Phe-Tyr-Lys-D-Arg-Trp-His-D-Lys-D-Lys-Glu-Arg-D-Tyr-Thr |
| Thr-Tyr-Arg-D-Lys-Trp-Tyr-Glu-Asp-D-Lys-D-Arg-His-Phe-D- Tyr-Gly-Val-Ile-D-His-Arg-Tyr-Lys-NH2 |
| Tyr-Asp-D-Lys-Tyr-Phe-D-Lys-D-Arg-Phe-Pro-D-Tyr-His-Lys |
| Tyr-D-His-Phe-D-Arg-Asp-Lys-D-Arg-His-Trp-D-His-Phe |
| Phe-Tyr-Lys-D-Arg-Trp-His-D-Lys-D-Lys-Glu-Arg-D-Tyr-Thr |
| Tyr-Asp-D-Lys-Tyr-Phe-D-Lys-D-Arg-Phe-Pro-D-Tyr-His-Lys |
| Glu-Arg-D-Lys-Tyr- D-Val-Phe- D-His-Trp-Arg-D-Gly-Tyr- Arg-D-Met-NH2 |
| Arg-D-Leu-D-Tyr-Phe-Lys-Glu- D-Lys-Arg-D-Trp-Lys- D-Phe- Tyr-D-Arg-Gly |
| Gly-Ala-Lys-Phe-D-Lys-Glu-Arg-Tyr-His-D-Arg-D-Arg-Asp- Tyr-Trp-D-His-Trp-His-D-Lys-Asp |
| Gly-Ala-Lys-Phe-D-Lys-Glu-Arg-Tyr-His-D-Arg-D-Arg-Asp- Tyr-Trp-D-His-Trp-His-D-Lys-Asp |

In one embodiment, the aromatic-cationic peptides of the present technology have a core structural motif of alternating aromatic and cationic amino acids. For example, the peptide may be a tetrapeptide defined by any of Formulas A to F set forth below:
Aromatic - Cationic - Aromatic - Cationic (Formula A)
Cationic - Aromatic - Cationic - Aromatic (Formula B)
Aromatic - Aromatic - Cationic - Cationic (Formula C)
Cationic - Cationic - Aromatic - Aromatic (Formula D)
Aromatic - Cationic - Cationic - Aromatic (Formula E)
Cationic - Aromatic - Aromatic - Cationic (Formula F)
wherein, Aromatic is a residue selected from the group consisting of: Phe (F), Tyr (Y), and Trp (W). In some embodiments, the Aromatic residue may be substituted with a saturated analog of an aromatic residue, *e.g.,* Cyclohexylalanine (Cha). In some embodiments, Cationic is a residue selected from the group consisting of: Arg (R), Lys (K), and His (H).

The amino acids of the aromatic-cationic peptides of the present technology can be any amino acid. As used herein, the term "amino acid" is used to refer to any organic molecule that contains at least one amino group and at least one carboxyl group. In some embodiments, at least one amino group is at the α position relative to the carboxyl group.

The amino acids may be naturally occurring. Naturally occurring amino acids include, for example, the twenty most common levorotatory (L,) amino acids normally found in mammalian proteins, *i.e*., alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophan, (Trp), tyrosine (Tyr), and valine (Val).

Other naturally occurring amino acids include, for example, amino acids that are synthesized in metabolic processes not associated with protein synthesis. For example, the amino acids ornithine and citrulline are synthesized in mammalian metabolism during the production of urea.

The peptides useful in the present technology can contain one or more non-naturally occurring amino acids. The non-naturally occurring amino acids may be (L-), dextrorotatory (D-), or mixtures thereof. In some embodiments, the peptide has no amino acids that are naturally occurring.

Non-naturally occurring amino acids are those amino acids that typically are not synthesized in normal metabolic processes in living organisms, and do not naturally occur in proteins. In certain embodiments, the non-naturally occurring amino acids useful in the present technology are also not recognized by common proteases.

The non-naturally occurring amino acid can be present at any position in the peptide. For example, the non-naturally occurring amino acid can be at the N terminus, the C-terminus, or at any position between the N-terminus and the C-terminus.

The non-natural amino acids may, for example, comprise alkyl, aryl, or alkylaryl groups. Some examples of alkyl amino acids include α-aminobutyric acid, β-aminobutyric acid, γ-aminobutyric acid, δ-aminovaleric acid, and ε-aminocaproic acid. Some examples of aryl amino acids include ortho-, meta, and para-aminobenzoic acid. Some examples of alkylaryl amino acids include ortho-, meta-, and para-aminophenyl acetic acid, and γ-phenyl-β-aminobutyric acid.

Non-naturally occurring amino acids also include derivatives of naturally occurring amino acids. The derivatives of naturally occurring amino acids may, for example, include the addition of one or more chemical groups to the naturally occurring amino acid.

For example, one or more chemical groups can be added to one or more of the 2', 3', 4', 5', or 6' position of the aromatic ring of a phenylalanine or tyrosine residue, or the 4', 5', 6', or 7' position of the benzo ring of a tryptophan residue. The group can be any chemical group that can be added to an aromatic ring. Some examples of such groups include branched or unbranched C₁-C₄ alkyl, such as methyl, ethyl, n-propyl, isopropyl, butyl, isobutyl, or t-butyl, C₁-C₄ alkyloxy (*i.e*., alkoxy), amino, C₁-C₄ alkylamino and C₁-C₄ dialkylamino (*e.g.,* methylamino, dimethylamino), nitro, hydroxyl, halo (*i.e.,* fluoro, chloro, bromo, or iodo). Some specific examples of non-naturally occurring derivatives of naturally occurring amino acids include norvaline (Nva), norleucine (Nle), and hydroxyproline (Hyp).

Another example of a modification of an amino acid in a peptide useful in the present methods is the derivatization of a carboxyl group of an aspartic acid or a glutamic acid residue of the peptide. One example of derivatization is amidation with ammonia or with a primary or secondary amine, e.g., methylamine, ethylamine, dimethylamine or diethylamine. Another example of derivatization includes esterification with, for example, methyl or ethyl alcohol.

Another such modification includes derivatization of an amino group of a lysine, arginine, or histidine residue. For example, such amino groups can be alkylated or acylated. Some suitable acyl groups include, for example, a benzoyl group or an alkanoyl group comprising any of the C₁-C₄ alkyl groups mentioned above, such as an acetyl or propionyl group.

In some embodiments, the non-naturally occurring amino acids are resistant, and in some embodiments insensitive, to common proteases. Examples of non-naturally occurring amino acids that are resistant or insensitive to proteases include the dextrorotatory (D-) form of any of the above-mentioned naturally occurring L-amino acids, as well as L- and/or D non-naturally occurring amino acids. The D-amino acids do not normally occur in proteins, although they are found in certain peptide antibiotics that are synthesized by means other than the normal ribosomal protein synthetic machinery of the cell, as used herein, the D-amino acids are considered to be non-naturally occurring amino acids.

In order to minimize protease sensitivity, the peptides useful in the methods of the present technology should have less than five, less than four, less than three, or less than two contiguous L-amino acids recognized by common proteases, irrespective of whether the amino acids are naturally or non-naturally occurring. In some embodiments, the peptide has only D-amino acids, and no L-amino acids.

If the peptide contains protease sensitive sequences of amino acids, at least one of the amino acids is a non-naturally-occurring D-amino acid, thereby conferring protease resistance. An example of a protease sensitive sequence includes two or more contiguous basic amino acids that are readily cleaved by common proteases, such as endopeptidases and trypsin. Examples of basic amino acids include arginine, lysine and histidine. In some embodiments, at least one of the amides in the peptide backbone is alkylated, thereby conferring protease resistance.

It is important that the aromatic-cationic peptides have a minimum number of net positive charges at physiological pH in comparison to the total number of amino acid residues in the peptide. The minimum number of net positive charges at physiological pH is referred to below as (pₘ). The total number of amino acid residues in the peptide is referred to below as (r).

The minimum number of net positive charges discussed below are all at physiological pH. The term "physiological pH" as used herein refers to the normal pH in the cells of the tissues and organs of the mammalian body. For instance, the physiological pH of a human is normally approximately 7.4, but normal physiological pH in mammals may be any pH from about 7.0 to about 7.8.

Typically, a peptide has a positively charged N-terminal amino group and a negatively charged C-terminal carboxyl group. The charges cancel each other out at physiological pH. As an example of calculating net charge, the peptide Tyr-Arg-Phe-Lys-Glu-His-Trp-Arg has one negatively charged amino acid (*i.e*., Glu) and four positively charged amino acids (*i.e.,* two Arg residues, one Lys, and one His). Therefore, the above peptide has a net positive charge of three.

In one embodiment, the aromatic-cationic peptides have a relationship between the minimum number of net positive charges at physiological pH (pₘ) and the total number of amino acid residues (r) wherein 3pₘ is the largest number that is less than or equal to r + 1. In this embodiment, the relationship between the minimum number of net positive charges (pₘ) and the total number of amino acid residues (r) is as follows:

| **TABLE 1. Amino acid number and net positive charges (3pₘ ≤ p+1)** | | | | | | | | | | | | | | | | | | |
|---|---|---|---|---|---|---|---|---|---|---|---|---|---|---|---|---|---|---|
| **(r)** | 3 | 4 | 5 | 6 | 7 | 8 | 9 | 10 | 11 | 12 | 13 | 14 | 15 | 16 | 17 | 18 | 19 | 20 |
| **(pₘ)** | 1 | 1 | 2 | 2 | 2 | 3 | 3 | 3 | 4 | 4 | 4 | 5 | 5 | 5 | 6 | 6 | 6 | 7 |

In another embodiment, the aromatic-cationic peptides have a relationship between the minimum number of net positive charges (pₘ) and the total number of amino acid residues (r) wherein 2pₘ is the largest number that is less than or equal to r + 1. In this embodiment, the relationship between the minimum number of net positive charges (pₘ) and the total number of amino acid residues (r) is as follows:

| **TABLE 2. Amino acid number and net positive charges (2pₘ ≤ p+1)** | | | | | | | | | | | | | | | | | | |
|---|---|---|---|---|---|---|---|---|---|---|---|---|---|---|---|---|---|---|
| **(r)** | 3 | 4 | 5 | 6 | 7 | 8 | 9 | 10 | 11 | 12 | 13 | 14 | 15 | 16 | 17 | 18 | 19 | 20 |
| **(pₘ)** | 2 | 2 | 3 | 3 | 4 | 4 | 5 | 5 | 6 | 6 | 7 | 7 | 8 | 8 | 9 | 9 | 10 | 10 |

In one embodiment, the minimum number of net positive charges (pₘ) and the total number of amino acid residues (r) are equal. In another embodiment, the peptides have three or four amino acid residues and a minimum of one net positive charge, or a minimum of two net positive charges, or a minimum of three net positive charges.

It is also important that the aromatic-cationic peptides have a minimum number of aromatic groups in comparison to the total number of net positive charges (pₜ). The minimum number of aromatic groups will be referred to below as (a). Naturally-occurring amino acids that have an aromatic group include the amino acids histidine, tryptophan, tyrosine, and phenylalanine. For example, the hexapeptide Lys-Gln-Tyr-D-Arg-Phe-Trp has a net positive charge of two (contributed by the lysine and arginine residues) and three aromatic groups (contributed by tyrosine, phenylalanine and tryptophan residues).

The aromatic-cationic peptides should also have a relationship between the minimum number of aromatic groups (a) and the total number of net positive charges at physiological pH (pₜ) wherein 3a is the largest number that is less than or equal to pₜ + 1, except that when pₜ is 1, a may also be 1. In this embodiment, the relationship between the minimum number of aromatic groups (a) and the total number of net positive charges (pₜ) is as follows:

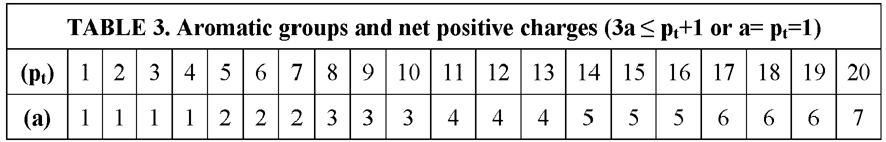

In another embodiment, the aromatic-cationic peptides have a relationship between the minimum number of aromatic groups (a) and the total number of net positive charges (pₜ) wherein 2a is the largest number that is less than or equal to pₜ + 1. In this embodiment, the relationship between the minimum number of aromatic amino acid residues (a) and the total number of net positive charges (pₜ) is as follows:

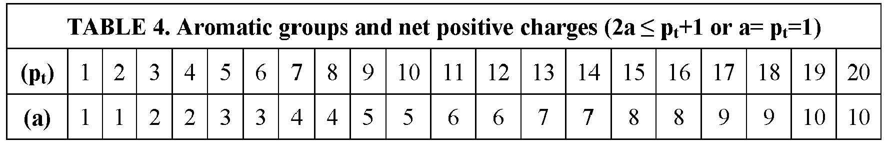

In another embodiment, the number of aromatic groups (a) and the total number of net positive charges (pt) are equal.

In some embodiments, carboxyl groups, especially the terminal carboxyl group of a C-terminal amino acid, are amidated with, for example, ammonia to form the C-terminal amide. Alternatively, the terminal carboxyl group of the C-terminal amino acid may be amidated with any primary or secondary amine. The primary or secondary amine may, for example, be an alkyl, especially a branched or unbranched C₁-C₄ alkyl, or an aryl amine. Accordingly, the amino acid at the C-terminus of the peptide may be converted to an amido, N-methylamido, N-ethylamido, N,N-dimethylamido, N,N-diethyl amido, N-methyl-N-ethylamido, N-phenylamido or N-phenyl-N-ethylamido group.

The free carboxylate groups of the asparagine, glutamine, aspartic acid, and glutamic acid residues not occurring at the C-terminus of the aromatic-cationic peptides of the present technology may also be amidated wherever they occur within the peptide. The amidation at these internal positions may be with ammonia or any of the primary or secondary amines described herein.

In one embodiment, the aromatic-cationic peptide useful in the methods of the present technology is a tripeptide having two net positive charges and at least one aromatic amino acid. In a particular embodiment, the aromatic-cationic peptide useful in the methods of the present technology is a tripeptide having two net positive charges and two aromatic amino acids.

In some embodiments, the aromatic-cationic peptide is a peptide having:
at least one net positive charge;
a minimum of four amino acids;
a maximum of about twenty amino acids;
a relationship between the minimum number of net positive charges (pₘ) and the total number of amino acid residues (r) wherein 3pₘ is the largest number that is less than or equal to r + 1; and a relationship between the minimum number of aromatic groups (a) and the total number of net positive charges (pₜ) wherein 2a is the largest number that is less than or equal to pₜ + 1, except that when a is 1, pₜ may also be 1.

In one embodiment, 2pₘ is the largest number that is less than or equal to r+1, and a may be equal to pₜ. The aromatic-cationic peptide may be a water-soluble peptide having a minimum of two or a minimum of three positive charges.

In one embodiment, the peptide comprises one or more non-naturally occurring amino acids, for example, one or more D-amino acids. In some embodiments, the C-terminal carboxyl group of the amino acid at the C-terminus is amidated. In certain embodiments, the peptide has a minimum of four amino acids. The peptide may have a total of about 6, a total of about 9, or a total of about 12 amino acids.

In one embodiment, the peptides have a tyrosine residue or a tyrosine derivative at the N-terminus (*i.e*., the first amino acid position). Suitable derivatives of tyrosine include 2'-methyltyrosine (Mmt); 2',6'-dimethyltyrosine (2'6'-Dmt); 3',5'-dimethyltyrosine (3'5'Dmt); N,2',6'-trimethyltyrosine (Tmt); and 2'-hydroxy-6'-methyltyrosine (Hmt).

In one embodiment, a peptide has the formula Tyr-D-Arg-Phe-Lys-NH₂. Tyr-D-Arg-Phe-Lys-NH₂ has a net positive charge of three, contributed by the amino acids tyrosine, arginine, and lysine and has two aromatic groups contributed by the amino acids phenylalanine and tyrosine. The tyrosine of Tyr-D-Arg-Phe-Lys-NH₂ can be a modified derivative of tyrosine such as in 2',6'-dimethyltyrosine to produce the compound having the formula 2',6'-Dmt-D-Arg-Phe-Lys-NH₂. 2',6'-Dmt-D-Arg-Phe-Lys-NH₂ has a molecular weight of 640 and carries a net three positive charge at physiological pH. 2',6'-Dmt-D-Arg-Phe-Lys-NH₂ readily penetrates the plasma membrane of several mammalian cell types in an energy-independent manner (Zhao et al., J. Pharmacol Exp Ther., 304:425-432, 2003).

Alternatively, in some embodiments, the aromatic-cationic peptide does not have a tyrosine residue or a derivative of tyrosine at the N-terminus (*i.e*., amino acid position 1). The amino acid at the N-terminus can be any naturally-occurring or non-naturally-occurring amino acid other than tyrosine. In one embodiment, the amino acid at the N-terminus is phenylalanine or its derivative. Exemplary derivatives of phenylalanine include 2'-methylphenylalanine (Mmp), 2',6'-dimethylphenylalanine (2',6'-Dmp), N,2',6'-trimethylphenylalanine (Tmp), and 2'-hydroxy-6'-methylphenylalanine (Hmp).

An example of an aromatic-cationic peptide that does not have a tyrosine residue or a derivative of tyrosine at the N-terminus is a peptide with the formula Phe-D-Arg-Phe-Lys-NH₂. Alternatively, the N-terminal phenylalanine can be a derivative of phenylalanine such as 2',6'-dimethylphenylalanine (2'6'-Dmp). In one embodiment, the amino acid sequence of 2',6'-Dmt-D-Arg-Phe-Lys-NH₂ is rearranged such that Dmt is not at the N-terminus. An example of such an aromatic-cationic peptide is a peptide having the formula of D-Arg-2'6'-Dmt-Lys-Phe-NH₂.

Suitable substitution variants of the peptides listed herein include conservative amino acid substitutions. Amino acids may be grouped according to their physicochemical characteristics as follows:
(a) Non-polar amino acids: Ala(A) Ser(S) Thr(T) Pro(P) Gly(G) Cys (C);
(b) Acidic amino acids: Asn(N) Asp(D) Glu(E) Gln(Q);
(c) Basic amino acids: His(H) Arg(R) Lys(K);
(d) Hydrophobic amino acids: Met(M) Leu(L) Ile(I) Val(V); and
(e) Aromatic amino acids: Phe(F) Tyr(Y) Trp(W) .

Substitutions of an amino acid in a peptide by another amino acid in the same group are referred to as a conservative substitution and may preserve the physicochemical characteristics of the original peptide. In contrast, substitutions of an amino acid in a peptide by another amino acid in a different group are generally more likely to alter the characteristics of the original peptide.

The amino acids of the peptides disclosed herein may be in either the L- or the D-configuration.

The peptides may be synthesized by any of the methods well known in the art. Suitable methods for chemically synthesizing the protein include, for example, those described by Stuart and Young in Solid Phase Peptide Synthesis, Second Edition, Pierce Chemical Company (1984), and in Methods Enzymol., 289, Academic Press, Inc., New York (1997).

### DMD

DMD is by far the most common childhood-onset muscular dystrophy, afflicting 1 in 3500 boys with an overall prevalence of 63 cases per million. One third of these cases are due to spontaneous mutations, while the rest are inherited in an X-linked manner. Gonadal mosaicism accounts for approximately 20% of new DMD cases.

DMD is caused by mutations in the dystrophin gene, which encodes a 427-kD protein that plays an integral role in the structural stability of the myofiber. Dystrophin is expressed mainly in smooth, cardiac, and skeletal muscles, with lower levels in the brain. The dystrophin gene consists of 79 exons and 8 promoters spread over 2.2 million base pairs of genomic DNA. Deletions and duplications account for approximately 59% and 5% of all dystrophin mutations, respectively. The remaining one third of the dystrophin mutations are composed of subexonic sequences, of which 34% are nonsense mutations, 33% are frameshifts, 29% are splice site mutations, and 4% are missense mutations.

### Pathogenesis

Dystrophin is expressed in muscle as a 427-kD protein that consists of 2 apposed globular heads with a flexible rod-shaped center that links the intracellular actin cytoskeleton to the extracellular matrix *via* the dystroglycan complex (or dystrophin-associated glycoprotein complex). The protein is organized into 4 structural domains including the amino-terminal actin-binding domain, a central rod domain, a cysteine-rich domain, and a carboxy-terminal domain. Its amino terminal end interacts with the subsarcolemmal actin filaments of myofibrils, while cysteine-rich domains of the carboxy-terminal end associate with beta-dystroglycan as well as elements of the sarcoglycan complex, all of which are contained within the sarcolemmal membrane. Beta-dystroglycan in turn anchors the entire complex to the basal lamina *via* laminin.

Deletions or duplications of the dystrophin gene that disturb the reading frame, including premature stop codons, produce a severely truncated, completely dysfunctional protein product or no protein at all. The functional loss of dystrophin protein initiates a cascade of events, including loss of other components of the dystrophin-associated glycoprotein complex, sarcolemmal breakdown with attendant calcium ion influx, high levels of creatine phosphokinase, phospholipase activation, and ultimately, myonecrosis.

Microscopic evaluation in the early stages of the disease reveals widespread myonecrosis with fiber splitting. Inflammatory cell infiltration of the necrotic fibers may be observed in particularly aggressive areas of muscle biopsies. Fibers that survive exhibit considerable variability and often demonstrate internal nuclei. As the disease progresses, dead muscle fibers are cleared away by macrophages and replaced by fatty and connective tissue elements, conveying a deceptively healthy appearance to the muscle (pseudohypertrophy), especially calves and forearms.

### Clinical Manifestations

Signs and symptoms of DMD include progressive proximal weakness with onset in the legs and pelvis, hyperlordosis with wide-based gait, hypertrophy of weak muscles, pseudohypertrophy (enlargement of calf and deltoid muscles with fat and fibrotic tissue), reduced muscle contractility on electrical stimulation in advanced stages of the disease, delayed motor milestones, progressive inability to ambulate, heel cord contractures, paralysis, fatigue, skeletal deformities including scoliosis, muscle fiber deformities, cardiomyopathy, congestive heart failure or arrhythmia, muscular atrophy, respiratory disorders, and absence of bladder or bowel dysfunction, sensory disturbance, or febrile illness. Weakness of skeletal muscle can contribute to cardiopulmonary complications. Scoliotic deformity from paraspinal muscle asymmetric atrophy impairs pulmonary and gastrointestinal function, predisposing individuals to pneumonia, respiratory failure, and poor nutrition. Smooth muscle dysfunction as a result of abnormal or absent dystrophin, along with inactivity, leads to gastrointestinal dysmotility, causing constipation and diarrhea.

The progression of DMD occurs in 5 stages: presymptomatic, early ambulatory, late ambulatory, early nonambulatory, and late nonambulatory.

### Diagnosis

DMD can be diagnosed in several ways. A clinical diagnosis may be made when a male child has progressive symmetrical muscle weakness. The symptoms manifest before age 5 years. If left untreated, the affected patients become wheelchair dependent before age 13 years.

Muscle biopsy is an important tool for quantifying the amount of muscle dystrophin as well as for detecting asymptomatic female carriers of DMD. Immunostaining of the muscle using antibodies directed against the rod domain, carboxy-terminals, and amino-terminals of dystrophin protein shows absence of the usual sarcolemmal staining in boys with DMD.

Genetic alterations in dystrophin can be detected using a variety of techniques in molecular biology such as direct sequencing, multiplex PCR amplification, Southern blot, quantitative PCR, multiplex amplifiable probe hybridization (MAPH), multiplex ligation-dependent probe (MLPA), denaturing high-performance liquid chromatography (dHPLC), single- stranded conformational polymorphism analysis with single condition amplification internal primers (SCAIP), or denaturing gradient gel electrophoresis.

For the remaining individuals, a combination of clinical findings, family history, blood concentration of creatine phosphokinase and muscle biopsy with dystrophin studies confirms the diagnosis. Creatine phosphokinase is normally present in high concentrations in the muscle cells. However, DMD patients exhibit creatine phosphokinase levels that are 50-100 times the reference range (as high as 20,000 mU/mL) during the early stages of the disease. Electromyography, electrocardiogram and echocardiogram, and lung monitoring tests may be used for confirmatory diagnosis of DMD.

### Current Treatments

There is no cure for DMD. Treatment is generally aimed at controlling the onset of symptoms to maximize the quality of life and include corticosteroids (*e.g.,* prednisone, deflazacort), Oxandrolone, ACE inhibitors (*e.g.,* Perindopril), P188 (Poloxamer 188), beta-blockers, diuretics, angiotensin receptor blockers (*e.g.,* Losartan), idebenone, alendronate, calcium with vitamin D, albuterol, dantrolene, pentoxifylline, carnitine, Coenzyme Q10, creatine, fish oil, green tea extracts, Vitamin E, PTC-124 (PTC Therapeutics Inc., South Plainfield, NJ), AVI-4658 phosphorodiamidate morpholino oligomer, azathioprine and cyclosporine. Unfortunately, chronic daily use of corticosteroids can cause weight gain, cataracts, osteoporosis, hypertension, diabetes, delayed puberty, stunted vertical growth, and behavioral/sleep issues.

Physical therapy is helpful to maintain muscle strength, flexibility, and function. Orthopedic appliances (such as braces and wheelchairs) may improve mobility and the ability for self-care. Form-fitting removable leg braces that hold the ankle in place during sleep can defer the onset of contractures. Appropriate respiratory support as the disease progresses is important.

### Regulators of Muscle Mass

Insulin like growth factor (IGF-1) is a positive regulator of muscle growth and has a profound effect on muscle precursor activation and proliferation. Upregulation of IGF-1 in the *mdx* mouse model for DMD exhibits functional improvement, restoration of muscle strength, and reduced fibrosis.

Regulation of myostatin is another alternative to preserving muscle mass and function. Myostatin is a member of the transforming growth factor beta (TGF-β) family and is a potent negative regulator of functional muscle mass. Overexpression of follistatin inhibits myostatin and has been shown to increase muscle mass in animal models. C. Colussi et al., Gene Therapy 15:1075-1076 (2008).

The utrophin gene at locus 6q24, located on the long arm of chromosome 6, shares structural and functional similarities with the dystrophin gene. Utrophin contains an actin-binding N-terminus, a triple coiled-coil repeat central region, and a C-terminus that consists of protein-protein interaction motifs which interact with dystroglycan protein components. Utrophin is expressed at the neuromuscular synapse and myotendinous junctions, where it participates in post-synaptic membrane maintenance and acetylcholine receptor clustering. Mouse studies suggest that utrophin may serve as a functional substitute for the dystrophin gene and therefore, may serve as a potential therapeutic alternative to DMD. Upregulation of the enzyme Galgt2 has been shown in animal models to be useful in stimulating the assembly of utrophin-glycoprotein complexes. Rodino-Klapac LR et al., Arch Neurol. 64(9): 1236-41 (2007).

Inhibition of calpains can also protect muscle mass. The activity of calpains can be thwarted by calpastatin, an endogenous inhibitor of calpains. The expression of calpastatins can be increased with α2-adrenergic agonists.

### Therapeutic Methods

The following discussion is presented by way of example only, and is not intended to be limiting.

One aspect of the present technology includes methods of treating DMD in a subject diagnosed as having, suspected as having, or at risk of having DMD. In therapeutic applications, compositions or medicaments comprising an aromatic-cationic peptide, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, hydrochloride salt, or trifluoroacetate salt, are administered to a subject suspected of, or already suffering from such a disease (such as, *e.g.,* subjects exhibiting elevated blood levels of creatine phosphokinase compared to a normal control subject or a subject harboring a genetic alteration that disrupts the production and/or function of dystrophin protein), in an amount sufficient to cure, or at least partially arrest, the symptoms of the disease, including its complications and intermediate pathological phenotypes in development of the disease.

Subjects suffering from DMD can be identified by any or a combination of diagnostic or prognostic assays known in the art. For example, typical symptoms of DMD include, but are not limited to, progressive proximal weakness with onset in the legs and pelvis, hyperlordosis with wide-based gait, hypertrophy of weak muscles, pseudohypertrophy (enlargement of calf and deltoid muscles with fat and fibrotic tissue), reduced muscle contractility on electrical stimulation in advanced stages of the disease, delayed motor milestones, progressive inability to ambulate, heel cord contractures, paralysis, fatigue, skeletal deformities including scoliosis, muscle fiber deformities, cardiomyopathy, congestive heart failure or arrhythmia, muscular atrophy, respiratory disorders, and absence of bladder or bowel dysfunction, sensory disturbance, or febrile illness.

In some embodiments, the subject may exhibit elevated blood levels of creatine phosphokinase compared to a normal control subject, which is measureable using techniques known in the art. In some embodiments, the subject may exhibit one or more genetic alterations that disrupt the production or function of dystrophin, which is involved in the assembly of the dystrophin-associated glycoprotein complex and is integral to the structural stability of the myofiber. In one particular embodiment, the genetic alteration is a deletion, duplication, frameshift, or nonsense mutation of dystrophin that is detectable using techniques known in the art.

In some embodiments, DMD subjects treated with the aromatic-cationic peptide will show amelioration or elimination of one or more of the following symptoms: progressive proximal weakness with onset in the legs and pelvis, hyperlordosis with wide-based gait, hypertrophy of weak muscles, pseudohypertrophy (enlargement of calf and deltoid muscles with fat and fibrotic tissue), reduced muscle contractility on electrical stimulation in advanced stages of the disease, delayed motor milestones, progressive inability to ambulate, heel cord contractures, paralysis, fatigue, skeletal deformities including scoliosis, muscle fiber deformities, cardiomyopathy, congestive heart failure or arrhythmia, muscular atrophy, and respiratory disorders.

In certain embodiments, DMD subjects treated with the aromatic-cationic peptide will show normalization of creatine phosphokinase blood levels by at least 5%, at least 10%, at least 25%, at least 50%, at least 75%, or at least 90% compared to untreated DMD subjects. In certain embodiments, DMD subjects treated with the aromatic-cationic peptide will show creatine phosphokinase blood levels that are similar to that observed in a normal control subject.

In some embodiments of the methods, DMD subjects treated with the aromatic-cationic peptide will show an increase in utrophin expression levels and/or activity by at least 5%, at least 10%, at least 25%, at least 50%, at least 75%, or at least 90% compared to an untreated DMD control subject. In certain embodiments, DMD subjects treated with the aromatic-cationic peptide will show an increase in IGF-1 expression levels and/or activity by at least 5%, at least 10%, at least 25%, at least 50%, at least 75%, or at least 90% compared to an untreated DMD control subject. In some embodiments of the methods, DMD subjects treated with the aromatic-cationic peptide will show an increase in follistatin expression levels and/or activity by at least 5%, at least 10%, at least 25%, at least 50%, at least 75%, or at least 90% compared to an untreated DMD control subject.

In some embodiments of the methods, DMD subjects treated with the aromatic-cationic peptide will show an increase in Galgt2 expression levels and/or activity by at least 5%, at least 10%, at least 25%, at least 50%, at least 75%, or at least 90% compared to an untreated DMD control subject. In some embodiments of the methods, DMD subjects treated with the aromatic-cationic peptide will show an increase in calpastatin expression levels and/or activity by at least 5%, at least 10%, at least 25%, at least 50%, at least 75%, or at least 90% compared to an untreated DMD control subject. In some embodiments of the methods, DMD subjects treated with the aromatic-cationic peptide will show a decrease in calpain expression levels and/or activity by at least 5%, at least 10%, at least 25%, at least 50%, at least 75%, or at least 90% compared to an untreated DMD control subject.

### Prophylactic Methods

In one aspect, the present technology provides a method for preventing or delaying the onset of DMD or symptoms of DMD in a subject at risk of having DMD. In some embodiments, the subject may exhibit one or more mutations in dystrophin, which is involved in the assembly of the dystrophin-associated glycoprotein complex.

Subjects at risk for elevated blood levels of creatine phosphokinase compared to a normal control subject or at risk for DMD can be identified by, *e.g.,* any or a combination of diagnostic or prognostic assays known in the art. In prophylactic applications, pharmaceutical compositions or medicaments of aromatic-cationic peptides, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, hydrochloride salt, or trifluoroacetate salt, are administered to a subject susceptible to, or otherwise at risk of a disease or condition such as *e.g.,* DMD, in an amount sufficient to eliminate or reduce the risk, or delay the onset of the disease, including biochemical, histologic and/or behavioral symptoms of the disease, its complications and intermediate pathological phenotypes presenting during development of the disease. Administration of a prophylactic aromatic-cationic peptide can occur prior to the manifestation of symptoms characteristic of the disease or disorder, such that the disease or disorder is prevented or, alternatively, delayed in its progression.

Subjects at risk for elevated blood levels of creatine phosphokinase compared to a normal control subject or DMD include, but are not limited to, subjects harboring mutations in dystrophin, which is involved in the assembly of the dystrophin-associated glycoprotein complex.

For therapeutic and/or prophylactic applications, a composition comprising an aromatic-cationic peptide, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, hydrochloride salt, or trifluoroacetate salt, is administered to the subject. In some embodiments, the peptide composition is administered one, two, three, four, or five times per day. In some embodiments, the peptide composition is administered more than five times per day. Additionally or alternatively, in some embodiments, the peptide composition is administered every day, every other day, every third day, every fourth day, every fifth day, or every sixth day. In some embodiments, the peptide composition is administered weekly, bi-weekly, tri-weekly, or monthly. In some embodiments, the peptide composition is administered for a period of one, two, three, four, or five weeks. In some embodiments, the peptide is administered for six weeks or more. In some embodiments, the peptide is administered for twelve weeks or more. In some embodiments, the peptide is administered for a period of less than one year. In some embodiments, the peptide is administered for a period of more than one year.

In some embodiments of the methods of the present technology, the aromatic-cationic peptide is administered daily for 1 week or more. In some embodiments of the methods of the present technology, the aromatic-cationic peptide is administered daily for 2 weeks or more. In some embodiments of the methods of the present technology, the aromatic-cationic peptide is administered daily for 3 weeks or more. In some embodiments of the methods of the present technology, the aromatic-cationic peptide is administered daily for 4 weeks or more. In some embodiments of the methods of the present technology, the aromatic-cationic peptide is administered daily for 6 weeks or more. In some embodiments of the methods of the present technology, the aromatic-cationic peptide is administered daily for 12 weeks or more.

In some embodiments, treatment with the aromatic-cationic peptide will prevent or delay the onset of one or more of the following symptoms: progressive proximal weakness with onset in the legs and pelvis, hyperlordosis with wide-based gait, hypertrophy of weak muscles, pseudohypertrophy (enlargement of calf and deltoid muscles with fat and fibrotic tissue), reduced muscle contractility on electrical stimulation in advanced stages of the disease, delayed motor milestones, progressive inability to ambulate, heel cord contractures, paralysis, fatigue, skeletal deformities including scoliosis, muscle fiber deformities, cardiomyopathy, congestive heart failure or arrhythmia, muscular atrophy, and respiratory disorders. In certain embodiments, the blood levels of creatine phosphokinase in DMD subjects treated with the aromatic-cationic peptide will resemble those observed in healthy controls.

In some embodiments, treatment with the aromatic-cationic peptide will result in an increase in utrophin expression levels and/or activity relative to that observed in an untreated DMD control subject. In certain embodiments, treatment with the aromatic-cationic peptide will result in an increase in IGF-1 expression levels and/or activity relative to that observed in an untreated DMD control subject. In some embodiments, treatment with the aromatic-cationic peptide will result in an increase in follistatin expression levels and/or activity relative to that observed in an untreated DMD control subject.

In some embodiments, treatment with the aromatic-cationic peptide will result in an increase in Galgt2 expression levels and/or activity relative to that observed in an untreated DMD control subject. In certain embodiments, treatment with the aromatic-cationic peptide will result in an increase in calpastatin expression levels and/or activity relative to that observed in an untreated DMD control subject. In other embodiments, treatment with the aromatic-cationic peptide will result in a reduction of calpain expression levels and/or activity relative to that observed in an untreated DMD control subject.

### Determination of the Biological Effect of the Aromatic-Cationic Peptide-Based Therapeutic

In various embodiments, suitable *in vitro* or *in vivo* assays are performed to determine the effect of a specific aromatic-cationic peptide-based therapeutic and whether its administration is indicated for treatment. In various embodiments, *in vitro* assays can be performed with representative animal models, to determine if a given aromatic-cationic peptide-based therapeutic exerts the desired effect on reducing or eliminating signs and/or symptoms of DMD. Compounds for use in therapy can be tested in suitable animal model systems including, but not limited to rats, mice, chicken, cows, monkeys, rabbits, and the like, prior to testing in human subjects. Similarly, for *in vivo* testing, any of the animal model system known in the art can be used prior to administration to human subjects. In some embodiments, *in vitro* or *in vivo* testing is directed to the biological function of 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, hydrochloride salt, or trifluoroacetate salt.

Animal models of DMD are known in the art, including, for example Golden retriever muscular dystrophy (GRMD) dogs, CXMDJ beagle dogs, hypertrophic feline muscular dystrophy (hfmd) cats, and *mdx* mice. *See* Spurney C., Muscle Nerve 44(1):8-19 (2011); Willmann R. et al., Neuromuscular Disorders 19:241-249 (2009); Partridge TA, FEBS J. 280(17):4177-86 (2013). Such models may be used to demonstrate the biological effect of aromatic-cationic peptides of the present technology, such as 2'6'-Dmt-D-Arg-Phe-Lys-NH₂, Phe-D-Arg-Phe-Lys-NH₂, or D-Arg-2'6'-Dmt-Lys-Phe-NH₂, in the prevention and treatment of conditions arising from disruption of a particular gene, and for determining what comprises a therapeutically effective amount of peptide in a given context.

### Modes of Administration and Effective Dosages

Any method known to those in the art for contacting a cell, organ or tissue with an aromatic-cationic peptide of the present technology, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, hydrochloride salt, or trifluoroacetate salt, may be employed. Suitable methods include *in vitro, ex vivo, or in vivo* methods. *In vivo* methods typically include the administration of an aromatic-cationic peptide, such as those described above, to a mammal, suitably a human. When used *in vivo* for therapy, the aromatic-cationic peptides, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, hydrochloride salt, or trifluoroacetate salt, are administered to the subject in effective amounts (*i.e.,* amounts that have desired therapeutic effect). The dose and dosage regimen will depend upon the degree of the infection in the subject, the characteristics of the particular aromatic-cationic peptide used, *e.g.,* its therapeutic index, the subject, and the subject's history.

The effective amount may be determined during pre-clinical trials and clinical trials by methods familiar to physicians and clinicians. An effective amount of a peptide useful in the methods may be administered to a mammal in need thereof by any of a number of well-known methods for administering pharmaceutical compounds. The peptide may be administered systemically or locally.

The peptide may be formulated as a pharmaceutically acceptable salt. The term "pharmaceutically acceptable salt" means a salt prepared from a base or an acid which is acceptable for administration to a patient, such as a mammal (*e.g.,* salts having acceptable mammalian safety for a given dosage regime). However, it is understood that the salts are not required to be pharmaceutically acceptable salts, such as salts of intermediate compounds that are not intended for administration to a patient. Pharmaceutically acceptable salts can be derived from pharmaceutically acceptable inorganic or organic bases and from pharmaceutically acceptable inorganic or organic acids. In addition, when a peptide contains both a basic moiety, such as an amine, pyridine or imidazole, and an acidic moiety such as a carboxylic acid or tetrazole, zwitterions may be formed and are included within the term "salt" as used herein. Salts derived from pharmaceutically acceptable inorganic bases include ammonium, calcium, copper, ferric, ferrous, lithium, magnesium, manganic, manganous, potassium, sodium, and zinc salts, and the like. Salts derived from pharmaceutically acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperadine, polyamine resins, procaine, purines, theobromine, triethylamine, trimethylamine, tripropylamine, tromethamine and the like. Salts derived from pharmaceutically acceptable inorganic acids include salts of boric, carbonic, hydrohalic (hydrobromic, hydrochloric, hydrofluoric or hydroiodic), nitric, phosphoric, sulfamic and sulfuric acids. Salts derived from pharmaceutically acceptable organic acids include salts of aliphatic hydroxyl acids (*e.g.,* citric, gluconic, glycolic, lactic, lactobionic, malic, and tartaric acids), aliphatic monocarboxylic acids (*e.g.,* acetic, butyric, formic, propionic and trifluoroacetic acids), amino acids (*e.g.,* aspartic and glutamic acids), aromatic carboxylic acids (*e.g.,* benzoic, p-chlorobenzoic, diphenylacetic, gentisic, hippuric, and triphenylacetic acids), aromatic hydroxyl acids (*e.g.,* o-hydroxybenzoic, p-hydroxybenzoic, 1-hydroxynaphthalene-2-carboxylic and 3-hydroxynaphthalene-2-carboxylic acids), ascorbic, dicarboxylic acids (*e.g.,* fumaric, maleic, oxalic and succinic acids), glucuronic, mandelic, mucic, nicotinic, orotic, pamoic, pantothenic, sulfonic acids (*e.g.,* benzenesulfonic, camphosulfonic, edisylic, ethanesulfonic, isethionic, methanesulfonic, naphthalenesulfonic, naphthalene-1,5-disulfonic, naphthalene-2,6-disulfonic and p-toluenesulfonic acids), xinafoic acid, and the like. In some embodiments, the salt is an acetate, tartrate, or trifluoroacetate salt.

The aromatic-cationic peptides described herein, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, hydrochloride salt, or trifluoroacetate salt, can be incorporated into pharmaceutical compositions for administration, singly or in combination, to a subject for the treatment or prevention of a disorder described herein. Such compositions typically include the active agent and a pharmaceutically acceptable carrier. As used herein the term "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

Pharmaceutical compositions are typically formulated to be compatible with its intended route of administration. Examples of routes of administration include parenteral (*e.g.,* intravenous, intradermal, intraperitoneal or subcutaneous), oral, inhalation, transdermal (topical), intraocular, iontophoretic, and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic. For convenience of the patient or treating physician, the dosing formulation can be provided in a kit containing all necessary equipment (*e.g.,* vials of drug, vials of diluent, syringes and needles) for a treatment course (*e.g.,* 7 days of treatment).

Pharmaceutical compositions suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, a composition for parenteral administration must be sterile and should be fluid to the extent that easy syringability exists. It should be stable under the conditions of manufacture and storage and must be preserved against the contaminating action of microorganisms such as bacteria and fungi.

The aromatic-cationic peptide compositions can include a carrier, which can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thiomerasol, and the like. Glutathione and other antioxidants can be included to prevent oxidation. In many cases, it will be advantageous to include isotonic agents, for example, sugars, polyalcohols such as mannitol, sorbitol, or sodium chloride in the composition. Prolonged absorption of the injectable compositions can be brought about by including in the composition an agent that delays absorption, for example, aluminum monostearate or gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, typical methods of preparation include vacuum drying and freeze drying, which can yield a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds can be delivered in the form of an aerosol spray from a pressurized container or dispenser, which contains a suitable propellant, *e.g.,* a gas such as carbon dioxide, or a nebulizer. Such methods include those described in U.S. Pat. No. 6,468,798.

Systemic administration of a therapeutic compound as described herein can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays. For transdermal administration, the active compounds are formulated into ointments, salves, gels, or creams as generally known in the art. In one embodiment, transdermal administration may be performed by iontophoresis.

A therapeutic protein or peptide can be formulated in a carrier system. The carrier can be a colloidal system. The colloidal system can be a liposome, a phospholipid bilayer vehicle. In one embodiment, the therapeutic peptide is encapsulated in a liposome while maintaining peptide integrity. One skilled in the art would appreciate that there are a variety of methods to prepare liposomes. (*See* Lichtenberg, et al., Methods Biochem. Anal., 33:337-462 (1988); Anselem, et al., Liposome Technology, CRC Press (1993)). Liposomal formulations can delay clearance and increase cellular uptake (*See* Reddy, Ann. Pharmacother., 34(7-8):915-923 (2000)). An active agent can also be loaded into a particle prepared from pharmaceutically acceptable ingredients including, but not limited to, soluble, insoluble, permeable, impermeable, biodegradable or gastroretentive polymers or liposomes. Such particles include, but are not limited to, nanoparticles, biodegradable nanoparticles, microparticles, biodegradable microparticles, nanospheres, biodegradable nanospheres, microspheres, biodegradable microspheres, capsules, emulsions, liposomes, micelles and viral vector systems.

The carrier can also be a polymer, *e.g.,* a biodegradable, biocompatible polymer matrix. In one embodiment, the therapeutic peptide can be embedded in the polymer matrix, while maintaining protein integrity. The polymer may be natural, such as polypeptides, proteins or polysaccharides, or synthetic, such as poly α-hydroxy acids. Examples include carriers made of, *e.g.,* collagen, fibronectin, elastin, cellulose acetate, cellulose nitrate, polysaccharide, fibrin, gelatin, and combinations thereof. In one embodiment, the polymer is poly-lactic acid (PLA) or copoly lactic/glycolic acid (PGLA). The polymeric matrices can be prepared and isolated in a variety of forms and sizes, including microspheres and nanospheres. Polymer formulations can lead to prolonged duration of therapeutic effect. (*See* Reddy, Ann. Pharmacother., 34(7-8):915-923 (2000)). A polymer formulation for human growth hormone (hGH) has been used in clinical trials. (*See* Kozarich and Rich, Chemical Biology, 2:548-552 (1998)).

Examples of polymer microsphere sustained release formulations are described in PCT publication WO 99/15154 (Tracy, et al.), U.S. Pat. Nos. 5,674,534 and 5,716,644 (both to Zale, et al.), PCT publication WO 96/40073 (Zale, et al.), and PCT publication WO 00/38651 (Shah, et al.). U.S. Pat. Nos. 5,674,534 and 5,716,644 and PCT publication WO 96/40073 describe a polymeric matrix containing particles of erythropoietin that are stabilized against aggregation with a salt.

In some embodiments, the therapeutic compounds are prepared with carriers that will protect the therapeutic compounds against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Such formulations can be prepared using known techniques. The materials can also be obtained commercially, e.g., from Alza Corporation and Nova Pharmaceuticals, Inc. Liposomal suspensions (including liposomes targeted to specific cells with monoclonal antibodies to cell-specific antigens) can also be used as pharmaceutically acceptable carriers. These can be prepared according to methods known to those skilled in the art, for example, as described in U.S. Pat. No. 4,522,811.

The therapeutic compounds can also be formulated to enhance intracellular delivery. For example, liposomal delivery systems are known in the art, see, *e.g.,* Chonn and Cullis, "Recent Advances in Liposome Drug Delivery Systems," Current Opinion in Biotechnology 6:698-708 (1995); Weiner, "Liposomes for Protein Delivery: Selecting Manufacture and Development Processes," Immunomethods, 4(3):201-9 (1994); and Gregoriadis, "Engineering Liposomes for Drug Delivery: Progress and Problems," Trends Biotechnol., 13(12):527-37 (1995). Mizguchi, et al., Cancer Lett., 100:63-69 (1996), describes the use of fusogenic liposomes to deliver a protein to cells both *in vivo* and *in vitro.*

Dosage, toxicity and therapeutic efficacy of any therapeutic agent can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit high therapeutic indices are advantageous. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds may be within a range of circulating concentrations that include the ED50 with little or no toxicity. The dosage may vary within this range depending upon the dosage form employed and the route of administration utilized. For any compound used in the methods, the therapeutically effective dose can be estimated initially from cell culture assays. A dose can be formulated in animal models to achieve a circulating plasma concentration range that includes the IC50 (*i.e*., the concentration of the test compound which achieves a half-maximal inhibition of symptoms) as determined in cell culture. Such information can be used to determine useful doses in humans accurately. Levels in plasma may be measured, for example, by high performance liquid chromatography.

Typically, an effective amount of the aromatic-cationic peptides, sufficient for achieving a therapeutic or prophylactic effect, ranges from about 0.000001 mg per kilogram body weight per day to about 10,000 mg per kilogram body weight per day. Suitably, the dosage ranges are from about 0.0001 mg per kilogram body weight per day to about 100 mg per kilogram body weight per day. For example dosages can be 1 mg/kg body weight or 10 mg/kg body weight every day, every two days or every three days or within the range of 1-10 mg/kg every week, every two weeks or every three weeks. In one embodiment, a single dosage of peptide ranges from 0.001-10,000 micrograms per kg body weight. In one embodiment, aromatic-cationic peptide concentrations in a carrier range from 0.2 to 2000 micrograms per delivered milliliter. An exemplary treatment regime entails administration once per day or once a week. In therapeutic applications, a relatively high dosage at relatively short intervals is sometimes required until progression of the disease is reduced or terminated, or until the subject shows partial or complete amelioration of symptoms of disease. Thereafter, the patient can be administered a prophylactic regime.

In some embodiments, a therapeutically effective amount of an aromatic-cationic peptide may be defined as a concentration of peptide at the target tissue of 10⁻¹² to 10⁻⁶ molar, e.g., approximately 10⁻⁷ molar. This concentration may be delivered by systemic doses of 0.001 to 100 mg/kg or equivalent dose by body surface area. The schedule of doses would be optimized to maintain the therapeutic concentration at the target tissue, such as by single daily or weekly administration, but also including continuous administration (e.g., parenteral infusion or transdermal application).

The skilled artisan will appreciate that certain factors may influence the dosage and timing required to effectively treat a subject, including but not limited to, the severity of the disease or disorder, previous treatments, the general health and/or age of the subject, and other diseases present. Moreover, treatment of a subject with a therapeutically effective amount of the therapeutic compositions described herein can include a single treatment or a series of treatments.

The mammal treated in accordance with the present methods can be any mammal, including, for example, farm animals, such as sheep, pigs, cows, and horses; pet animals, such as dogs and cats; laboratory animals, such as rats, mice and rabbits. In some embodiments, the mammal is a human.

### Combination Therapy with Aromatic-Cationic Peptides

In some embodiments, the aromatic-cationic peptides, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, hydrochloride salt, or trifluoroacetate salt, may be combined with one or more additional therapies for the prevention or treatment of DMD. Additional therapeutic agents include, but are not limited to, corticosteroids, Oxandrolone, ACE inhibitors, P188 (Poloxamer 188), beta-blockers, diuretics, angiotensin receptor blockers (ARBs), idebenone, alendronate, calcium with vitamin D, albuterol, dantrolene, pentoxifylline, carnitine, Coenzyme Q10, creatine, fish oil, green tea extracts, Vitamin E, PTC-124 (PTC Therapeutics Inc., South Plainfield, NJ), AVI-4658 phosphorodiamidate morpholino oligomer, azathioprine and cyclosporine.

In some embodiments, the corticosteroids are selected from the group consisting of prednisone and deflazacort. In some embodiments, the ACE inhibitors are selected from the group consisting of captopril, alacepril, lisinopril, imidapril, quinapril, temocapril, delapril, benazepril, cilazapril, trandolapril, enalapril, ceronapril, fosinopril, imadapril, mobertpril, perindopril, ramipril, spirapril, randolapril and pharmaceutically acceptable salts of such compounds. In some embodiments, the ARBs are selected from the group consisting of losartan, candesartan, valsartan, eprosartan, telmisartan, and irbesartan.

In one embodiment, an additional therapeutic agent is administered to a subject in combination with an aromatic cationic peptide, such that a synergistic therapeutic effect is produced. For example, administration of the peptide with one or more additional therapeutic agents for the prevention or treatment of DMD will have greater than additive effects in the prevention or treatment of the disease. Therefore, lower doses of one or more of any individual therapeutic agent may be used in treating or preventing DMD, resulting in increased therapeutic efficacy and decreased side-effects. In some embodiments, the aromatic-cationic peptide is administered in combination with one or more additional therapeutic agents selected from the group consisting of corticosteroids, Oxandrolone, ACE inhibitors, P188 (Poloxamer 188), beta-blockers, diuretics, ARBs, idebenone, alendronate, calcium with vitamin D, albuterol, dantrolene, pentoxifylline, carnitine, Coenzyme Q10, creatine, fish oil, green tea extracts, Vitamin E, PTC-124 (PTC Therapeutics Inc., South Plainfield, NJ), AVI-4658 phosphorodiamidate morpholino oligomer, azathioprine and cyclosporine, such that a synergistic effect in the prevention or treatment of DMD results.

In any case, the multiple therapeutic agents may be administered in any order or even simultaneously. If simultaneously, the multiple therapeutic agents may be provided in a single, unified form, or in multiple forms (by way of example only, either as a single pill or as two separate pills). One of the therapeutic agents may be given in multiple doses, or both may be given as multiple doses. If not simultaneous, the timing between the multiple doses may vary from more than zero weeks to less than four weeks. In addition, the combination methods, compositions and formulations are not to be limited to the use of only two agents.

### EXAMPLES

The present technology is further illustrated by the following examples, which should not be construed as limiting in any way. For each of the examples below, any aromatic-cationic peptide described herein could be used. By way of example, but not by limitation, the aromatic-cationic peptide used in the example below could be 2'6'-Dmt-D-Arg-Phe-Lys-NH₂, Phe-D-Arg-Phe-Lys-NH₂, or D-Arg-2'6'-Dmt-Lys-Phe-NH₂ or any one or more of the peptides shown in Tables A, B, C, D, and/or E.

### Example 1 - Use of Aromatic-Cationic Peptides in the Treatment of DMD in Humans

This Example demonstrates the use of aromatic-cationic peptides, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, hydrochloride salt, or trifluoroacetate salt, in the treatment of DMD.

### Methods

Subjects suspected of having or diagnosed as having DMD receive daily administrations of 1%, 5% or 10% solution of aromatic-cationic peptide, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, hydrochloride salt, or trifluoroacetate salt, alone or in combination with one or more additional therapeutic agents for the treatment or prevention of DMD. Peptides and/or additional therapeutic agents selected from the group consisting of: corticosteroids, Oxandrolone, ACE inhibitors, P188 (Poloxamer 188), beta-blockers, diuretics, angiotensin receptor blockers (ARBs), idebenone, alendronate, calcium with vitamin D, albuterol, dantrolene, pentoxifylline, carnitine, Coenzyme Q10, creatine, fish oil, green tea extracts, Vitamin E, PTC-124 (PTC Therapeutics Inc., South Plainfield, NJ), AVI-4658 phosphorodiamidate morpholino oligomer, azathioprine and cyclosporine, are administered orally, topically, systemically, intravenously, subcutaneously, intraperitoneally, or intramuscularly according to methods known in the art. Dosages of the one or more additional therapeutic agents will range between 0.1 mg/kg to 50 mg/kg. Subjects will be evaluated weekly for the presence and/or severity of signs and symptoms associated with DMD, including, but not limited to, *e.g.,* progressive proximal weakness with onset in the legs and pelvis, hyperlordosis with wide-based gait, hypertrophy of weak muscles, pseudohypertrophy (enlargement of calf and deltoid muscles with fat and fibrotic tissue), reduced muscle contractility on electrical stimulation in advanced stages of the disease, delayed motor milestones, progressive inability to ambulate, heel cord contractures, paralysis, fatigue, skeletal deformities including scoliosis, muscle fiber deformities, cardiomyopathy, congestive heart failure or arrhythmia, muscular atrophy, and respiratory disorders. Treatments are maintained until such a time as one or more signs or symptoms of DMD are ameliorated or eliminated.

### Results

It is predicted that subjects suspected of having or diagnosed as having DMD and receiving therapeutically effective amounts of aromatic-cationic peptide, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, or trifluoroacetate salt will display reduced severity or elimination of symptoms associated with DMD. It is also expected that DMD subjects treated with the aromatic-cationic peptide will show normalization of creatine phosphokinase blood levels by at least 5% compared to the untreated DMD controls. It is further expected that administration of 2'6'-Dmt-D-Arg-Phe-Lys-NH₂, Phe-D-Arg-Phe-Lys-NH₂, or D-Arg-2'6'-Dmt-Lys-Phe-NH₂ in combination with one or more additional therapeutic agents will have synergistic effects in this regard compared to that observed in subjects treated with the aromatic-cationic peptides or the additional therapeutic agents alone.

These results will show that aromatic-cationic peptides, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, or trifluoroacetate salt are useful in the treatment of DMD. These results will show that aromatic-cationic peptides, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, or trifluoroacetate salt are useful in ameliorating one or more of the following symptoms: progressive proximal weakness with onset in the legs and pelvis, hyperlordosis with wide-based gait, hypertrophy of weak muscles, pseudohypertrophy (enlargement of calf and deltoid muscles with fat and fibrotic tissue), reduced muscle contractility on electrical stimulation in advanced stages of the disease, delayed motor milestones, progressive inability to ambulate, heel cord contractures, paralysis, fatigue, skeletal deformities including scoliosis, muscle fiber deformities, cardiomyopathy, congestive heart failure or arrhythmia, muscular atrophy, and respiratory disorders. Accordingly, the peptides are useful in methods comprising administering aromatic-cationic peptides to a subject in need thereof for the treatment of DMD.

### Example 2 - Use of Aromatic-Cationic Peptides in the Prevention of DMD in Humans

This example demonstrates the use of aromatic-cationic peptides, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, hydrochloride salt, or trifluoroacetate salt, in the prevention of DMD.

### Methods

Subjects at risk of having DMD receive daily administrations of 1%, 5% or 10% solution of aromatic-cationic peptide, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, hydrochloride salt, or trifluoroacetate salt, alone or in combination with one or more additional therapeutic agents for the treatment or prevention of DMD. Peptides and/or additional therapeutic agents selected from the group consisting of: corticosteroids, Oxandrolone, ACE inhibitors, P188 (Poloxamer 188), beta-blockers, diuretics, angiotensin receptor blockers (ARBs), idebenone, alendronate, calcium with vitamin D, albuterol, dantrolene, pentoxifylline, carnitine, Coenzyme Q10, creatine, fish oil, green tea extracts, Vitamin E, PTC-124 (PTC Therapeutics Inc., South Plainfield, NJ), AVI-4658 phosphorodiamidate morpholino oligomer, azathioprine and cyclosporine, are administered orally, topically, systemically, intravenously, subcutaneously, intraperitoneally, or intramuscularly according to methods known in the art. Dosages of the one or more additional therapeutic agents will range between 0.1 mg/kg to 50 mg/kg. Subjects will be evaluated weekly for the presence and/or severity of signs and symptoms associated with DMD, including, but not limited to, e.g., progressive proximal weakness with onset in the legs and pelvis, hyperlordosis with wide-based gait, hypertrophy of weak muscles, pseudohypertrophy (enlargement of calf and deltoid muscles with fat and fibrotic tissue), reduced muscle contractility on electrical stimulation in advanced stages of the disease, delayed motor milestones, progressive inability to ambulate, heel cord contractures, paralysis, fatigue, skeletal deformities including scoliosis, muscle fiber deformities, cardiomyopathy, congestive heart failure or arrhythmia, muscular atrophy, and respiratory disorders.

### Results

It is predicted that subjects at risk of having or diagnosed as having DMD and receiving therapeutically effective amounts of aromatic-cationic peptide, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, or trifluoroacetate salt will display delayed onset of DMD, or prevention of onset of DMD. It is also expected that the blood levels of creatine phosphokinase in DMD subjects treated with the aromatic-cationic peptide will resemble healthy controls. It is further expected that administration of 2'6'-Dmt-D-Arg-Phe-Lys-NH₂, Phe-D-Arg-Phe-Lys-NH₂, or D-Arg-2'6'-Dmt-Lys-Phe-NH₂ in combination with one or more additional therapeutic agents will have synergistic effects in this regard compared to that observed in subjects treated with aromatic-cationic peptides or the additional therapeutic agents alone.

These results will show that aromatic-cationic peptides, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, or trifluoroacetate salt are useful in the prevention of DMD. These results will show that aromatic-cationic peptides, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, or trifluoroacetate salt are useful in preventing or delaying the onset of one or more of the following symptoms: progressive proximal weakness with onset in the legs and pelvis, hyperlordosis with wide-based gait, hypertrophy of weak muscles, pseudohypertrophy (enlargement of calf and deltoid muscles with fat and fibrotic tissue), reduced muscle contractility on electrical stimulation in advanced stages of the disease, delayed motor milestones, progressive inability to ambulate, heel cord contractures, paralysis, fatigue, skeletal deformities including scoliosis, muscle fiber deformities, cardiomyopathy, congestive heart failure or arrhythmia, muscular atrophy, and respiratory disorders.

Accordingly, the peptides are useful in methods comprising administering aromatic-cationic peptides to a subject in need thereof for the prevention of DMD.

### Example 3 - Use of Aromatic-Cationic Peptides in the Treatment of DMD in a Mouse Model

This Example demonstrates the use of aromatic-cationic peptides, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, hydrochloride salt, or trifluoroacetate salt, in the treatment of DMD in a mouse model.

3-4 week-old male C57BL/10ScSn-Dmd*^{mdx}*/J (*mdx*) and C57BL/10ScSn (wild-type) mice will be housed in an individually vented cage system with a 12-hour light- dark cycle and will receive standard mouse chow and purified water *ad libitum.* The *mdx* mice are randomly assigned to either a sham (untreated) group or aromatic-cationic peptide-treated group (mdx-peptide). Mice in the *mdx*-peptide group are given a daily intraperitoneal injection of peptide (1-16 mg/kg), whereas the sham group will receive a daily intraperitoneal injection of vehicle. All treatments will commence a few days before the beginning of the exercise protocol, and will continue until the day of sacrifice.

*Exercise Protocol and in Vivo Studies.* Wild-type and *mdx* (both sham and peptide-treated) mice will undergo a 30-min run on a horizontal treadmill (Columbus Instruments, Columbus, OH) at 12 m/min, twice a week, for 4 to 8 weeks (Granchelli *et al*., 2000). The training protocol starts at the mouse age of 3 to 4 weeks. About half of the *mdx* mice will show an avoidance behavior with respect to exercise, with a higher tendency to fatigue, and will have to be gently stimulated, or left resting, to complete the 30-min running session. This behavior is absent in wild-type animals. Every week, all the exercised mice are monitored for body weight. The force for exercised mice (both wild-type controls and *mdx*) is evaluated before each training section by means of a grip strength meter (Columbus Instruments). For this measurement, the mice are allowed to grasp a triangular ring connected to a force transducer, which is then gently pulled away until the grip is broken. The transducer will save the force value at this point, which is a measure of the maximal resistance the animal can use with its forelimbs. Five measurements are taken from each animal within 2 min, and the maximum values are used for statistical analysis. At the end of the 4th week of exercise, the animals will be sacrificed.

*Histology.* Tibialis anterior (TA) muscle is rapidly rinsed in normal physiological solution and immediately frozen in isopentane cooled in liquid nitrogen. The samples are stored at -80°C until used for histological determination. Frozen muscle is cut into 10-mm-thick sections with a cryostat taken from the midpoint of the muscle body and stained with hematoxylin-eosin. A semiquantitative approach will be used to evaluate histopathological indexes and to allow statistical analysis. In particular, within a comparable number of fibers per section taken for each condition, the number of cell showing necrosis, centronucleation, and/or belonging to inflammatory infiltrates is calculated.

*Results.* It is predicted that the untreated *mdx* mice will exhibit weaker forelimb strength compared to age-matched wild-type controls. It is also predicted that the TA muscles of *mdx* mice will be histologically distinguishable from wild type - more disorganized structure due to the presence of necrosis, centronucleated fibers, regenerating fibers, and infiltration of mononuclear inflammatory cells. It is also anticipated that the forelimb strength and the histology of the TA muscles of peptide-treated *mdx* mice will resemble that observed in age-matched wild-type controls.

These results will show that aromatic-cationic peptides, such as 2'6'-Dmt-D-Arg-Phe-Lys-NH₂, Phe-D-Arg-Phe-Lys-NH₂, or D-Arg-2'6'-Dmt-Lys-Phe-NH₂, or a pharmaceutically acceptable salt thereof, such as acetate, tartrate, or trifluoroacetate salt are useful in the treatment of DMD in a mouse model. Accordingly, the peptides are useful in methods comprising administering aromatic-cationic peptides to a subject in need thereof for the treatment of DMD.

### Example 4- Use of Aromatic-Cationic Peptides in the Treatment of DMD in a DBA/2J-mdx Mouse Model

This Example demonstrates the use of Phe-D-Arg-Phe-Lys-NH₂, in the treatment of DMD in a mouse model.

*Methods:* DBA/2J-mdx (also known as D2.B10-DMD^{mdx}/2J) mice exhibit several of the human characteristics of DMD myopathology (lower hind limb muscle weight, fewer myofibers, increased fibrosis and fat accumulation, and muscle weakness). Left ventricular mitochondrial H₂O₂ emission (measured by spectrofluorometry; Amplex UltraRed fluorophore) and respiration (measured by high-resolution respirometry; Oroboros Oxygraph) were assessed in permeabilized muscle fiber bundles (PmFB) from DBA/2J-mdx mice (DMD mice) that were about 4 weeks of age, DBA/2J wildtype mice (wt mice) that were about 4 weeks of age, DMD mice treated with daily subcutaneous injections of Phe-D-Arg-Phe-Lys-NH₂ (5mg/kg) for 12 weeks (treated DMD mice), and DMD mice treated with daily subcutaneous injections of (0.9%) for 12 weeks (untreated DMD mice). H₂O₂ emission was measured in the presence of physiological ADP (25µM). ADP is a suppressor of H₂O₂ emission during respiration and is affected by the phosphate-shuttling activity of mitochondrial creatine kinase (mtCK). The effect of mtCK was assessed by saturating creatine (Cr) *in vitro.*

*Results:* At 4 weeks of age, ADP-suppression of Complex I-supported H₂O₂ (5mM Pyruvate/2mM Malate) was impaired in DMD mice in the presence of Cr (+Cr: 36+/-4% of emission at 0µM ADP in DMD mice as compared to 12+/-1% of emission at 0µM ADP in wt mice when expressed per O₂ consumed; p<0.01). At 4 weeks, ADP-suppression of Complex I-supported H₂O₂ was also observed in DMD mice in the absence of Cr (-Cr: 36+/-4 % of emission at 0µM ADP in DMD mice as compared to 17+/-2% of emission at 0µM ADP in wt mice when expressed per O₂ consumed; p<0.01). This data shows that H₂O₂ emission is elevated in DMD mice independent of mtCK-dependent phosphate shuttling.

Additionally, at 4 weeks of age, ADP, complex I-supported respiration was lower in DMD mice as compared to wt mice in both +Cr (30.8+/-5.0 pmol•s⁻¹•mg wet wt⁻¹ in DMD mice as compared to 54.2+/-10.2 pmol•s⁻¹•mg wet wt⁻¹ in wt mice, p<0.05) and -Cr (35.0+/- 1.2 pmol•s⁻¹•mg wet wt⁻¹ in DMD mice as compared to 52.0+/-5.4 pmol•s⁻¹•mg wet wt⁻¹ in wt mice, p<0.01).

After treatment of DMD mice for 12 weeks with Phe-D-Arg-Phe-Lys-NH₂ or saline, treated DMD mice had greater ADP-suppression of Complex I-supported H₂O₂ as compared to untreated DMD mice in +Cr (12+/-1% (treated DMD mice) vs 15+/-0% (untreated DMD mice) of emission at 0µM ADP/O₂ consumed, p<0.01). There were no differences in -Cr (40+/-11% (treated DMD mice) vs 42+/-13% (untreated DMD mice) of 0µM ADP/O₂ consumed). This data shows that treatment with Phe-D-Arg-Phe-Lys-NH₂ improved mtCK-dependent ADP suppression of H₂O₂ emission in treated treated DMD mice as compared to untreated DMD mice.

Treatment with Phe-D-Arg-Phe-Lys-NH₂ did not change respiration in treated DMD mice as compared to untreated DMD mice in +Cr (111.4 +/- 12.0 pmol•s⁻¹•mg wet wt⁻¹ (treated DMD mice) vs 82.1 +/- 11.2 pmol•s⁻¹•mg wet wt⁻¹ (untreated DMD mice), p=0.10) or -Cr (59.6 +/- 7.4 (treated DMD mice) vs 65.3 +/- 6.8 pmol•s⁻¹•mg wet wt⁻¹(untreated DMD mice)).

These results show that Phe-D-Arg-Phe-Lys-NH₂ is useful in the treatment of DMD. The results show that Phe-D-Arg-Phe-Lys-NH₂ increases the ability of mtCK to mediate ADP-suppression of H₂O₂ in treated DMD mice as compared to untreated DMD mice. Accordingly, aromatic-cationic peptides disclosed herein are useful for the treatment of DMD.

### EQUIVALENTS

The present technology is not to be limited in terms of the particular embodiments described in this application, which are intended as single illustrations of individual aspects of the present technology. Many modifications and variations of this present technology can be made without departing from its spirit and scope, as will be apparent to those skilled in the art. Functionally equivalent methods and apparatuses within the scope of the present technology, in addition to those enumerated herein, will be apparent to those skilled in the art from the foregoing descriptions. Such modifications and variations are intended to fall within the scope of the appended claims. The present technology is to be limited only by the terms of the appended claims, along with the full scope of equivalents to which such claims are entitled. It is to be understood that this present technology is not limited to particular methods, reagents, compounds compositions or biological systems, which can, of course, vary. It is also to be understood that the terminology used herein is for the purpose of describing particular embodiments only, and is not intended to be limiting.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, *etc.* As a non-limiting example, each range discussed herein can be readily broken down into a lower third, middle third and upper third, *etc.* As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

All patents, patent applications, provisional applications, and publications referred to or cited herein are incorporated by reference in their entirety, including all figures and tables, to the extent they are not inconsistent with the explicit teachings of this specification.

Other embodiments are set forth within the following claims.

## Claims

1. A peptide D-Arg-2',6'-Dmt-Lys-Phe-NH₂, or a pharmaceutically acceptable salt thereof, for use in treating Duchenne muscular dystrophy (DMD) in a human subject in need thereof.

2. The peptide for the use of claim 1, wherein the peptide is administered daily for 6 weeks or more.

3. The peptide for the use of claim 1 or claim 2, wherein the peptide is administered daily for 12 weeks or more.

4. The peptide for the use of any one of claims 1-3, wherein the subject has been diagnosed as having DMD, optionally wherein the subject has signs or symptoms of DMD

5. The peptide for the use of claim 4, wherein the signs or symptoms of DMD comprise one or more of progressive proximal weakness with onset in the legs and pelvis, hyperlordosis with wide-based gait, hypertrophy of weak muscles, pseudohypertrophy (enlargement of calf and deltoid muscles with fat and fibrotic tissue), reduced muscle contractility on electrical stimulation in advanced stages of the disease, delayed motor milestones, progressive inability to ambulate, heel cord contractures, paralysis, fatigue, skeletal deformities including scoliosis, muscle fiber deformities, cardiomyopathy, congestive heart failure or arrhythmia, muscular atrophy, and respiratory disorders.

6. The peptide for the use of any one of claims 1-5, wherein the peptide is administered orally, topically, systemically, intravenously, subcutaneously, transdermally, iontophoretically, intranasally, intraperitoneally, or intramuscularly.

7. The peptide for the use of any one of claims 1-6, further comprising separately, sequentially or simultaneously administering an additional therapeutic agent to the subject.

8. The peptide for the use of claim 7, wherein the additional therapeutic agent is selected from the group consisting of: corticosteroids, Oxandrolone, ACE inhibitors, P188 (Poloxamer 188), beta-blockers, diuretics, angiotensin receptor blockers (ARBs), idebenone, alendronate, calcium with vitamin D, albuterol, dantrolene, pentoxifylline, carnitine, Coenzyme Q10, creatine, fish oil, green tea extracts, Vitamin E, PTC-124, AVI-4658 phosphorodiamidate morpholino oligomer, azathioprine and cyclosporine.

9. The peptide for the use of any one of claims 1-8, wherein the pharmaceutically acceptable salt comprises acetate, tartrate or trifluoroacetate salt.

10. The peptide for the use of any one of claims 1-9, wherein the subject harbors a deletion, duplication, frameshift, or nonsense mutation in the dystrophin gene.
